(19) Europäisches Patentamt
European Patent Office
Office européen des brevets

(11) EP 4 062 889 A1

(12) **EUROPEAN PATENT APPLICATION**
published in accordance with Art. 153(4) EPC

(43) Date of publication:
28.09.2022 Bulletin 2022/39

(21) Application number: 19958276.8

(22) Date of filing: 31.12.2019

(51) International Patent Classification (IPC):
**A61H 7/00** (2006.01)  **A61F 7/00** (2006.01)

(52) Cooperative Patent Classification (CPC):
**A61F 7/00**

(86) International application number:
**PCT/CN2019/130792**

(87) International publication number:
**WO 2021/134605 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: Huawei Technologies Co., Ltd.
**Longgang**
**Shenzhen, Guangdong 518129 (CN)**

(72) Inventors:
• YANG, Hui
  **Shenzhen, Guangdong 518129 (CN)**

• ZHANG, Huimin
  **Shenzhen, Guangdong 518129 (CN)**
• LIU, Chang
  **Shenzhen, Guangdong 518129 (CN)**
• LI, Xu
  **Shenzhen, Guangdong 518129 (CN)**

(74) Representative: **Isarpatent**
**Patent- und Rechtsanwälte Barth**
**Charles Hassa Peckmann & Partner mbB**
**Friedrichstrasse 31**
**80801 München (DE)**

(54) **INTELLIGENT CONTROL APPARATUS AND CONTROL METHOD THEREFOR, AND INTELLIGENT WEARABLE DEVICE**

(57)    This application provides an intelligent control apparatus (100, 100', 900, 1620), including: a control circuit (120, 120', 910, 1622), a transceiver circuit (130, 210, 940, 1623), and a detection and heating circuit (110, 110', 300, 300', 1621). The detection and heating circuit (110, 110', 300, 300', 1621) includes a detection and heating material (310, 310', 1001, 1501, 1621). When the detection heating material (310, 310', 1001, 1501, 1621) works in a detection mode, the detection and heating circuit (110, 110', 300, 300', 1621) obtains a bioelectric signal of a user by using the detection and heating material (310, 310', 1001, 1501, 1621). The transceiver circuit (130, 210, 940, 1623) sends detection information corresponding to the bioelectric signal to a target device (200) and receives first instruction information corresponding to the detection information. The control circuit (120, 120', 910, 1622) instructs, according to the first instruction information, the detection heating circuit (110, 110', 300, 300', 1621) to switch a working mode of the detection and heating material (310, 310', 1001, 1501, 1621) to a heating mode; and instructs the detection and heating circuit (110, 110', 300, 300', 1621) to determine heating duration and/or a heating temperature of the detecting and heating material (310, 310', 1001, 1501, 1621). The apparatus (100, 100', 900, 1620) may be used in the field of artificial intelligence. Through control of the control circuit (120, 120', 910, 1622), the detection heating material (310, 310', 1001, 1501, 1621) can have both detection and heating functions. Intelligent perception of a state of the user is implemented by detecting of the bioelectric signal.

FIG. 1

EP 4 062 889 A1

## Description

### TECHNICAL FIELD

This application relates to the field of electronic devices, and more specifically, to an intelligent control apparatus and a control method therefor, and an intelligent wearable device.

### BACKGROUND

With development of society, changes of living and working habits and increase of pressure that are brought by modernization cause negative physiological and mental states of people, such as fatigue, anxiety, depression, poor sleep quality, stress, and tension. In an existing solution, approaches such as heating and vibration massage are usually used to reduce or eliminate the foregoing negative states of people, for example, mitigate fatigue, mitigate anxiety, improve sleep quality, and mitigate stress, so as to improve the states of people.

To meet higher requirements of people, currently, there is an improvement apparatus that allows a user to manually select different levels based on requirements of the user. For example, in a heating eye mask, different temperature levels are set, the temperature levels correspond to different heating temperatures, and the user may manually select a temperature level to control a heating temperature, thereby mitigating eye fatigue to different extents, or achieving functions such as mitigating pressure and promoting sleep. For another example, in a vibration massage wearable device, different vibration levels are set, the vibration levels correspond to different massage strength, and the user may select a vibration level based on a requirement and/or preference of the user, thereby improving a state of the user to different extents. However, in the foregoing solution, the user needs to make selections autonomously, and intelligent services cannot be provided for the user.

Therefore, how to intelligently improve the state of the user and improve user comfort and use experience is an urgent problem to be resolved.

### SUMMARY

This application provides an intelligent control apparatus to intelligently improve a state of a user and improve user comfort and use experience.

According to a first aspect, an intelligent control apparatus is provided. The apparatus includes a control circuit, a transceiver circuit, and a detection and heating circuit. The detection and heating circuit includes a detection and heating material. The detection and heating circuit controls, according to an instruction of the control circuit, switching of a working mode of the detection and heating material. When the working mode of the detection and heating material is a detection mode, the detection and heating circuit obtains first detection information of a user by using the detection and heating material, where the first detection information is obtained based on a first bioelectric signal. The transceiver circuit sends second detection information to a target device, where the second detection information is obtained based on the first detection information. The transceiver circuit is further configured to receive first instruction information sent by the target device and corresponding to the second detection information, and send the first instruction information to the control circuit. The control circuit instructs, according to the first instruction information, the detection and heating circuit to switch the working mode of the detection and heating material to a heating mode, and instructs, according to the first instruction information, the detection and heating circuit to control heating duration and/or a heating temperature of the detection and heating material in the heating mode.

The apparatus may be applied to the field of artificial intelligence. Control by the control circuit enables the detection and heating material to have both detection and heating functions, and intelligent perception of a state of the user is implemented through bioelectric signal detection. In the technical solution of this application, the control circuit and the detection and heating circuit control the detection and heating material to detect a bioelectric signal, and perform heating intervention on the user based on the detected bioelectric signal, thereby implementing a function of intelligently improving the state of the user. In addition, the detection and heating material has two functions: bioelectric signal detection and heating. Therefore, when the intelligent control apparatus is applied to an intelligent wearable device, a material at a same position in the intelligent wearable device is enabled to have both a conventional conducting function and a function of a conventional heating intervention apparatus. On one hand, complexity of the device is reduced. On the other hand, for a position at which an abnormality is detected, intervention can be performed at the position to improve the state of the user at the position more accurately.

Optionally, detecting bioelectric signals of the user may be detecting bioelectric signals of different parts of a body of the user. For example, the bioelectric signals include an electroencephalogram signal of a head, an electro-oculogram signal of an eye, and an electromyography signal of a face, and may further include an electrocardiosignal, and an electrical signal of a limb part. In other words, the bioelectric signal in this embodiment of this application may include one or more of the foregoing bioelectric signals. In this embodiment of this application, it is assumed that the foregoing electroencephalogram signal, electro-oculogram signal, and electromyography signal are included. However, it should be understood that this is only an example for description and does not constitute a limitation on the bioelectric signals.

It should be understood that when the detection and heating material works in the detection mode, the detection and heating material has a same function as a conventional electrode, that is, used to detect a bioelectric signal; or when the detection and heating material works in the heating mode, the detection and heating material also has a same function as a conventional heating intervention apparatus, that is, used to perform intervention on the user through heating, thereby improving the state of the user.

The conventional intervention apparatus may include various intervention apparatuses for heating, massage, music playback, video playback, and the like that can intervene in a physiological state and/or mental state of the user. For example, a state such as fatigue or soreness may be mitigated through heating massage or vibration massage, and a brain of the user may be relaxed through listening to music or viewing a video, thereby mitigating tension of the user. When detecting a bioelectric signal, the conventional electrode may obtain a bioelectric signal at a detection electrode by measuring a potential difference between the detection electrode and a reference electrode.

Optionally, when the detection and heating material works in the detection mode, a bioelectric signal of the user may be obtained by measuring a potential difference between a to-be- measured position and a reference position. When the detection and heating material works in the heating mode, the detection and heating material may be energized to heat the detection and heating material, and a voltage and/or current of an applied electrical signal are/is controlled to control a temperature of the detection and heating material.

Optionally, in consideration of comfort, when the intelligent control apparatus provided in this embodiment of this application is used for the intelligent wearable device, the detection and heating material may be woven in a base material of the intelligent wearable device to make a fabric that has both detection and heating functions.

Optionally, the detection and heating material may be made of one or more of materials such as silver plating, silver chloride plating, and graphene fiber.

Optionally, the detection and heating material may be machined from a combination of a conducting material and a heat conducting material. The conducting material is used to detect the first bioelectric signal of the user, and send the detected bioelectric signal to the control circuit. The heat conducting material is used for heating. It should be understood that, in this example, the conducting material may be a material having only conductivity, and the heat conducting material may be a material having only heat conductivity. For example, the conducting material may be an inorganic water-absorbing and water-retaining material, an organic material of a hydrophilic functional group, an alloy material, or a metal material, and the heat conducting material may be a heat conducting silica gel, a heat conducting silicone, a carbon-based material, an alloy material, or a metal material. These material components undergo fiber processing, micro-nano processing, and the like. For example, the material is attached to a fiber surface or a foam tissue surface, or the material is machined onto another substrate through micro-nano processing. In this way, the detection and heating material is machined from the conducting material and the heat conducting material.

For further example, the conducting material and the heat conducting material may be woven into the detection and heating material in a weaving manner. It should be understood that the two materials may also be made into the detection and heating material through combined machining in other manners, where the conducting material is used for bioelectric signal detection, and the heat conducting material is used for heating. Optionally, a cross-weaving manner may be used. However, it should be understood that another weaving manner such as front-back weaving may be used.

It should be understood that both the conducting material and the heat conducting material may be made of a mixture of one or more materials. To be specific, the conducting material may be made of one or more materials having conductivity, and the heat conducting material may also be made of one or more materials having heat conductivity. It should also be understood that the conducting material may be a material having only conductivity, or may be a material having both conductivity and heat conductivity. Similarly, the heat conducting material may be a material having only heat conductivity, or may be a material having both conductivity and heat conductivity.

Optionally, the detection and heating material is connected to a circuit module in the detection and heating circuit, where the conducting material is connected to a detection circuit in the detection and heating circuit, and the heat conducting material is connected to a heating circuit in the detection and heating circuit. For example, the conducting material and the heat conducting material may be connected to the corresponding circuits by using wires.

Optionally, when the detection and heating material works in the detection mode, the conducting material may detect a bioelectric signal of the user, and transmit the bioelectric signal to the detection circuit by using a wire, and the detection circuit may obtain the bioelectric signal and perform corresponding processing.

Optionally, when the detection and heating material works in the heating mode, the heating circuit may control heating duration and/or a heating temperature of the heat conducting material according to an instruction of the control circuit.

It should be noted that the conducting material in the detection and heating material in the foregoing example is used only for bioelectric signal detection and not used for heating; and the heat conducting material is used only for heating, and not used for bioelectric signal detection. In other words, even if the conducting material is a material having both conductivity and heat conductivity, the conducting material is only enabled to implement a conducting function and used for bioelectric signal detection; and even if the heat conducting material is a material having both conductivity and heat conductivity, the heat conducting material is only enabled to implement a heat conducting function and used for heating.

In the foregoing example, the conducting material and the heat conducting material are disposed at a same position after certain machining, so that the material at the same position has both the conducting function and the heat conducting function. However, in different working modes, only one of the conducting material and the heat conducting material generally works. In other words, only the conducting material works in the detection mode, and only the heat conducting material works in the heating mode. When the detection and heating material is used for intelligently perceiving the state of the user and intelligently improving the state of the user, after an abnormal bioelectric signal is detected by using a conducting material at a position, heating intervention can be performed by using a heat conducting material at the same position, to improve a body part more accurately and improve user experience.

Optionally, for example, through weaving or sticking, the detection and heating material may be further made of one or more materials having both conductivity and heat conductivity. For example, a silver plating material may be woven into the detection and heating material, or a silver plating material and a graphene fiber material may be woven into the detection and heating material for both heating and bioelectric signal detection. It should be noted that, in this example, the detection and heating material may be used for both heating and bioelectric signal detection.

Optionally, one or more materials having both conductivity and heat conductivity may be woven into the detection and heating material, and the detection and heating material is connected to the detection and heating circuit. Optionally, the detection and heating material may be woven in a cross-weaving manner, or may be woven in another manner such as front-back weaving. Alternatively, the detection and heating material may be made in a manner other than weaving.

Optionally, the detection and heating material may be connected to the detection circuit and the heating circuit in the detection and heating circuit by using wires. It should be noted that only a part of the detection and heating material may be connected to both the detection circuit and the heating circuit. For example, other parts may be used only as ordinary fabrics and not connected to any circuit. In other words, only a part of the material may be enabled to work in two modes.

In one case, when the detection and heating circuit sets, according to an instruction of the control circuit, the detection and heating material to work in the detection mode, a bioelectric signal of the user may be obtained by using the detection and heating material and transmitted to the detection circuit by using a wire, and the detection circuit may obtain the bioelectric signal and perform corresponding processing to obtain corresponding detection information.

Optionally, to prevent a bioelectric signal detection process from being affected, an electrical signal applied to the detection and heating material by the heating circuit in this case may be shielded by the detection circuit, and the connection/disconnection of the detection circuit and the heating circuit may be controlled by setting a switch and controlling on/off of the switch.

In another case, when the detection and heating circuit sets, according to an instruction of the control circuit, the detection and heating material to work in the heating mode, the detection and heating circuit may apply an electrical signal such as a current or a voltage to the detection and heating material by using the heating circuit, so that the detection and heating material performs heating, and the detection and heating circuit may control strength of the electrical signal, to control the heating temperature of the detection and heating material.

Optionally, the detection and heating circuit may further control the heating duration of the detection and heating material by controlling duration of applying an electrical signal to the detection and heating material. For example, a timer may be set. After the detection and heating circuit connects the electrical signal applied to the material, timing is started. After a time specified by the timer expires, the electrical signal is disconnected. In this case, the heating duration of the material can be controlled.

Optionally, the foregoing two detection and heating materials may be used in combination. In other words, the detection and heating material includes a material part having both conductivity and heat conductivity, and this material part is connected to both the detection circuit and the heating circuit; and the detection and heating material further includes a material part having only conductivity or having only heat conductivity, and in this material part, the conducting material is connected to the detection circuit, and the heat conducting material is connected to the heating circuit. It should be noted that in each of the foregoing examples of the detection and heating material, there may be a material used only as an ordinary fabric and not connected to any circuit.

With reference to the first aspect, in some implementations of the first aspect, the detection and heating circuit may be configured to: when the detection and heating material is controlled to work in the detection mode, block an electrical signal that is applied to the detection and heating material and used to control heating of the detection and heating material; or when the detection and heating material works in the heating mode, control the heating temperature of the detection and heating material by controlling an electrical signal applied to the detection and heating material.

Optionally, a switch may be set on the detection circuit and the heating circuit, and the electrical signal applied to the detection and heating material can be controlled by controlling on/off of the switch. In other words, in the detection mode, the detection and heating material is connected to the detection circuit (that is, there is an electrical connection), and is disconnected from the heating circuit (that is, there is no electrical connection); or in the heating mode, the detection and heating material is connected to the heating circuit (that is, there is an electrical connection), and is disconnected from the detection circuit (that is, there is no electrical connection).

Optionally, when the detection and heating material works in the detection mode, the electrical signal connected by using the heating circuit may be blocked. To be specific, in the detection mode, the electrical signal applied to the detection and heating material by the heating circuit is blocked; or in the heating mode, the electrical signal is applied to the detection and heating material by the heating circuit to heat the detection and heating material, and the temperature of the detection and heating material is controlled by controlling the strength of the applied electrical signal. It should be understood that, in this example, the electrical signal applied to the detection and heating material by the heating circuit may not be disconnected in another form such as a switch, but in the detection mode, a blocking circuit is set to block the electrical signal applied to the detection and heating material by the heating circuit.

Optionally, during bioelectric signal detection, a direct current or working frequency suppression module may be used to block a direct current signal used to control heating, so that no interference is caused to the bioelectric signal detection process. When bioelectric signal detection is not performed, the electrical signal used to control heating of the detection and heating material is applied to heat the detection and heating material, and the temperature of the detection and heating material is controlled by the strength of the applied electrical signal. The direct current or working frequency suppression module can block direct current and working frequency interference from other circuits, for example, block 50 Hz working frequency interference, or for another example, block a direct current signal that is applied to the detection and heating material and used for heating, so that no interference is caused to bioelectric signal detection. In this case, the direct current or working frequency suppression module implements a circuit protection function, and may be considered as a circuit protection apparatus.

It can be learned that, in this embodiment of this application, under control of the detection and heating circuit, the detection and heating material is enabled to work in two working modes, that is, the detection and heating material has both the bioelectric signal detection function and the heating function. To implement switching between the two working modes, the detection and heating circuit may be controlled by the control circuit.

With reference to the first aspect, in some implementations of the first aspect, the first detection information may be a bioelectric signal obtained after the first bioelectric signal is processed by using the detection circuit. The second detection information may be the first detection information itself, or certain processing such as decomposition or feature extraction may be performed on the first detection information to obtain the second detection information.

Optionally, a signal decomposition module may be set to decompose the first detection information. Optionally, the first detection information may be decomposed by using a blind signal separation method, to obtain a decomposed signal of the first detection information. For example, an ICA method is used to decompose the first detection information to obtain source signal components of the first detection information, where the components may correspond to an electroencephalogram signal, an electro-oculogram signal, an electromyography signal, and the like. In this way, the electroencephalogram signal, the electro-oculogram signal, and the electromyography signal are obtained through decomposition.

Optionally, the decomposed signal of the first detection information, for example, the electroencephalogram signal, the electro-oculogram signal, or the electromyography signal obtained through decomposition, may be used as the second detection information.

Optionally, the control circuit may further perform feature extraction on the first detection information to obtain a feature signal corresponding to the first detection information, or the control circuit may perform feature extraction on the decomposed signal of the first detection information to obtain a feature signal of the decomposed signal of the first detection information, and use the feature signal as the second detection information.

Optionally, when the control circuit performs feature extraction on the decomposed signal of the first detection information, a method of energy sum ratio analysis in a frequency domain analysis method may be used to extract the feature signal of the decomposed signal such as the electroencephalogram signal, the electro-oculogram signal, or the electromyography signal obtained by decomposing the first detection information.

It can be learned that the second detection information may be a bioelectric signal obtained after the bioelectric signal is processed, or may be a decomposed signal obtained after the processed bioelectric signal is decomposed, or may be a feature signal obtained after feature extraction is performed on the decomposed signal.

It should be understood that, what is available between the transceiver circuit and the target device may be a wired transmission communication mode, or may be an electrical connection using a wire, or may be a wireless transmission communication mode. Alternatively, the transceiver circuit may be a transceiver, an interface circuit, a communications unit, a communications module, a transceiver unit, a transceiver module, or the like. Details are not described herein.

Optionally, a temperature controller, a temperature control circuit, a temperature control module, or the like (hereinafter collectively referred to as a temperature controller) may also be set, so that the heating temperature of the detection and heating material can be stabilized within a temperature range in the heating mode. In other words, heating may be performed within a floating range of a specified heating temperature. For example, when the heating temperature is set to 40°C, the temperature controller may be used to stabilize the heating temperature within a range of 40±0.5°C. It should be understood that a structure of the temperature controller is not limited, but may be selected as required by a person skilled in the art. The setting of the floating range is merely an example, and is not limited either. Details are not described herein. It should also be understood that the temperature controller may be disposed in the control circuit, or may be disposed in the detection and heating circuit, or may be independent of the control circuit and the detection and heating circuit.

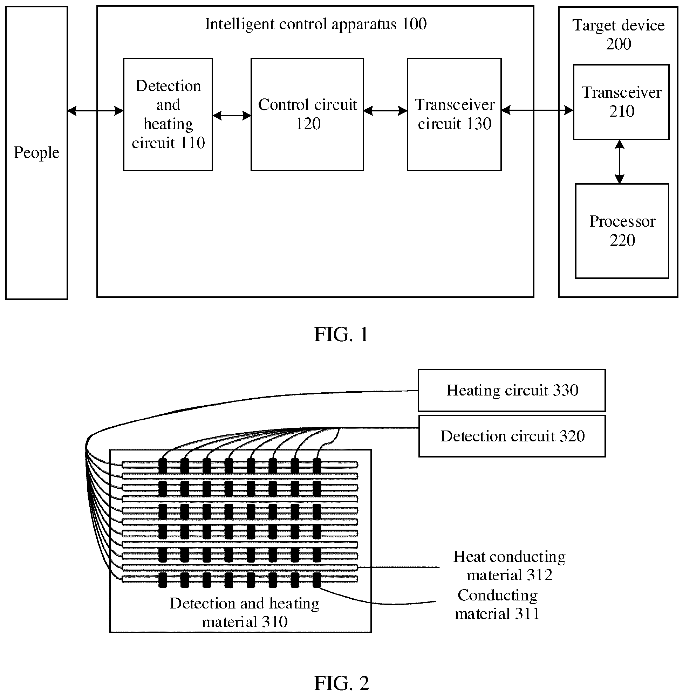

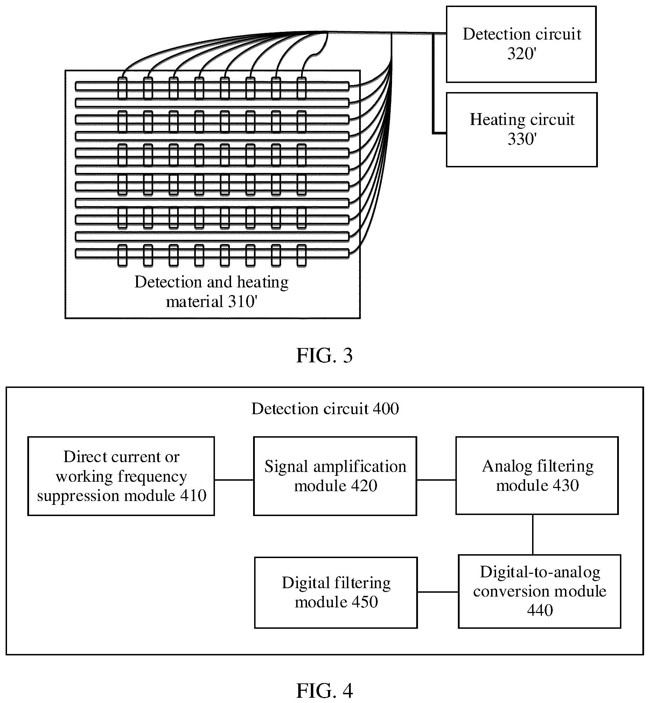

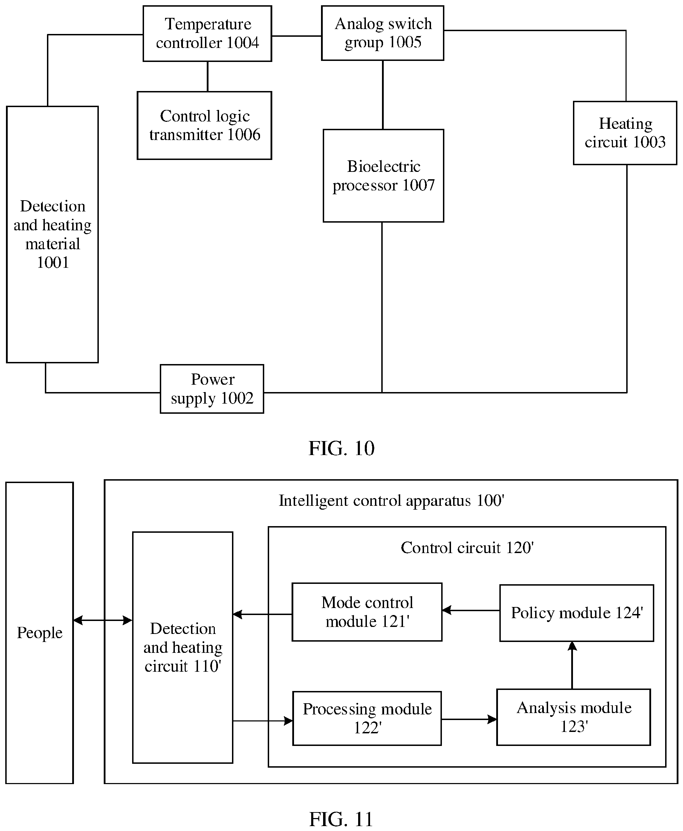

The temperature controller, also referred to as an automatic temperature regulator, is usually implemented by using a circuit. A core of this circuit is to set a temperature threshold by using a heat-sensitive element, to trigger turn-on or turn-off of a switch. Temperature adjustments may also be divided into fine-granularity level adjustments. For example, there are three levels: Temperature T1 > Temperature T2 > Temperature T3. If a currently specified temperature level is the temperature T3, and a temperature sensor in the temperature controller detects that a current temperature Tc is lower than the temperature T3, that is, Tc < T3, a temperature controller control switch is connected to a heating module (for example, the heating circuit shown in FIG. 2 or FIG. 3). When the temperature Tc detected by the temperature sensor is higher than or equal to the temperature T2, that is, Tc ≥ T2, the temperature controller switch is connected to a detection module (for example, the detection circuit shown in FIG. 2 or FIG. 3 or the detection circuit shown in FIG. 4), and a bioelectric signal detected by the detection module is transmitted to a processing apparatus (for example, the target device shown in FIG. 1 or the control circuit shown in FIG. 11), so that the processing apparatus can obtain a new policy for adjusting the state of the user, for example, obtain a new temperature level and apply the new level to the temperature controller.

Optionally, the temperature controller may be electronic or mechanical.

Optionally, the temperature controller may be programmed to use a program to control and send various signals to the heating circuit of the conducting and heating material, to achieve an objective of controlling the connection/disconnection of the heating circuit and implement a function of a switch.

Optionally, in addition to controlling the heating mode and a parameter according to the received first instruction information from the target device, the control circuit may further control switching of the two working modes autonomously. For example, it may be specified that when a detected bioelectric signal is not within a preset range, the working mode is switched to the heating mode, and fixed heating duration is set, for example, set to 5 minutes (min); after 5 minutes, the working mode is switched to the detection mode, and detection is performed again; if a detected bioelectric signal is within the preset range, the process is stopped; if a detected bioelectric signal is not within the preset range, the process of heating for 5 minutes is repeated, and the process is not stopped until a detected bioelectric signal is within the preset range. It should be understood that this implementation may be considered as a process of alternate detection and heating, where the process is not stopped until a value is within the preset range. However, there is also another implementation. For example, in a process of repeating heating for fixed duration, the heating temperature may be adjusted based on a difference between a detected bioelectric signal and the preset range.

For further example, for example, it may be specified that when the detected bioelectric signal is within the preset value range, heating intervention is not performed; or both the heating temperature and duration are set to be relatively small, and when the detected bioelectric signal is not within the preset value range, heating intervention is performed. In addition, the heating duration and/or temperature may be adjusted based on the difference between the detected bioelectric signal and the preset range.

With reference to the first aspect, in some implementations of the first aspect, the first instruction information is used to indicate an improvement solution determined by the target device based on the second detection information, where the improvement solution includes the heating duration and/or the heating temperature of the detection and heating material working in the heating mode; the transceiver circuit receives the first instruction information, and sends the first instruction information to the control circuit; and the control circuit instructs, according to the first instruction information, the detection and heating circuit to switch the working mode of the detection and heating material to the heating mode, and the control circuit further instructs, according to the first instruction information, the detection and heating circuit to perform the improvement solution.

Optionally, the intelligent control apparatus may further include a vibration massage apparatus. In this case, when determining the improvement solution, the intelligent control apparatus not only indicates the heating duration and/or the heating temperature of the detection and heating material, but also indicates vibration intervention duration and/or vibration strength of the vibration massage apparatus, or the like.

Optionally, the improvement solution may be determined by the target device based on the second detection information and a state category of the user.

Optionally, a fatigue category of the user may be further determined by the target device based on an established fatigue category classification model and the second detection information.

With reference to the first aspect, in some implementations of the first aspect, when the duration of the detection and heating material working in the heating mode is greater than or equal to a first preset value, the control circuit instructs the detection and heating circuit to switch the working mode of the detection and heating material to the detection mode; the detection and heating circuit obtains third detection information of the user by using the detection and heating material, where the third detection information is obtained based on a second bioelectric signal of the user; and the transceiver circuit sends fourth detection information to the target device, where the fourth detection information is obtained based on the third detection information. Therefore, the target device can update a user state category classification model based on the fourth detection information.

It should be understood that the first preset value may be a value less than the heating duration of the heating mode, and this is equivalent to pausing heating in a heating process to detect the second bioelectric signal. Alternatively, the first preset value may be a value greater than or equal to the heating duration of the heating mode, and this is equivalent to detecting the second bioelectric signal after the heating ends.

For example, the duration of performing heating is originally set to 10 minutes, the first preset value is 5 minutes, the detection and heating material pauses heating after performing heating for 5 minutes according to a mitigation solution, and works in the detection mode instead, to detect the second bioelectric signal of the user, so that the target device can update the state category classification model based on the second bioelectric signal.

It should be noted that, in actual application, the intelligent control apparatus provided in this embodiment of this application may further set an initial mode or a power-on mode. For example, the power-on mode may be set to the detection mode. When the user is using the intelligent control apparatus, detection is performed on the user after power-on, and then the heating temperature and/or the heating duration are/is determined based on the detected bioelectric signal. For another example, the initial mode or the power-on mode may alternatively be set to a mode that can be manually selected and switched by the user. For example, a mode selection module may be set, so that the user can autonomously select the initial mode or the power-on mode.

According to a second aspect, an intelligent control system is provided. The system includes any intelligent control apparatus in the first aspect and a target device. The target device receives second detection information from the intelligent control apparatus, and determines a state category of a user based on the second detection information; and the target device determines first instruction information based on the state category, and sends the first instruction information to the intelligent control apparatus. The first instruction information is used to instruct a detection and heating material in the intelligent control apparatus to switch to a heating mode and indicate duration and/or a heating temperature of the detection and heating material working in the heating mode. The state category is used to indicate a state of the user, and the state of the user may include at least one of a fatigue state, an anxiety state, a sleep state, a tension state, and a depression state.

In the system, the intelligent control apparatus cooperates with the target device to implement intelligent perception of the state of the user and intelligent improvement of the state of the user. The intelligent system may be applied to the field of artificial intelligence. Control by the intelligent control apparatus and a process of confirming the state category of the user and an improvement solution by the target device can implement the intelligent perception and intelligent improvement of the state of the user.

Optionally, the target device may determine the state category of the user based on the second detection information, determine a corresponding improvement solution based on the state category, and generate first instruction information corresponding to the improvement solution, where the improvement solution includes the heating duration and/or the heating temperature of the detection and heating material working in the heating mode; and the target device sends the first instruction information to the intelligent control apparatus, to instruct the intelligent control apparatus to perform the improvement solution.

In the process of determining the state category of the user based on the second detection information, the target device may perform different processing based on different cases of the second detection information.

When the second detection information is first detection information, the target device decomposes the second detection information to obtain a decomposed signal, then performs feature extraction on the decomposed signal to obtain a feature signal, and then determines the state category of the user based on the feature signal. When the second detection information is a decomposed signal of the first detection information, the target device performs feature extraction on the second detection information to obtain a feature signal, and then determines the state category of the user based on the feature signal. When the second detection information is a feature signal of the first detection information, the target device directly determines the state category of the user based on the second detection information.

Optionally, when determining the state category of the user based on the extracted feature signal, the target device may analyze and determine the state category by using the following method.

It should be noted that the state category of the user is used to indicate the state of the user, and the state of the user may be various mental states or physiological states such as the fatigue state, the anxiety state, the sleep state, the depression state, a stress state, or the tension state. For example, the state category may be classified into a normal state, a slightly abnormal state, a moderately abnormal state, and a severely abnormal state. When corresponding to a state, for example, corresponding to the fatigue state, the fatigue state category may be classified into non-fatigue, general fatigue, and great fatigue.

Optionally, the target device may be configured to compare a bioelectric signal (for example, an electroencephalogram signal, an electro-oculogram signal, or an electromyography signal) of the user with a normal value or a preset value, definean abnormal level based on a difference, and further determine the state category of the user. For example, both the electroencephalogram signal and the electromyography signal determined based on the detected bioelectric signal differ greatly from the normal value; therefore, it is inferred that the user is in the severely abnormal state, such as great fatigue. For ease of understanding, the following uses the fatigue state of the user as an example for description. Therefore, it may be understood that in the following method, the fatigue state may also be replaced with another state, for example, the user state such as the anxiety state or the depression state.

Optionally, the fatigue category of the user may be classified, for example, classified into non-fatigue, general fatigue, and great fatigue. Further, a fatigue degree of the user may be classified, for example, classified into slight fatigue, moderate fatigue, or severe fatigue. Alternatively, a fatigue degree of the user may be classified into levels. For example, the fatigue degree is classified into the following levels: 1-non-fatigue, 2-general fatigue, and 3-great fatigue.

It should be understood that the fatigue category, the fatigue degree, and the fatigue degree level are all descriptions of the fatigue state of the user, and belong to different descriptions of a same thing. In this embodiment of this application, the fatigue category is used as an example for description.

Optionally, the target device may be configured to set value ranges of corresponding bioelectric signals of different fatigue categories, compare the second detection information with a preset range corresponding to the second detection information, and determine the fatigue category of the user based on a comparison result. Because a first bioelectric signal corresponds to the first detection information and the first detection information corresponds to the second detection information, it may be considered that a determined correspondence exists between the first bioelectric signal and the second detection information.

With reference to the second aspect, in some implementations of the second aspect, the target device may be configured to determine the state category of the user based on the second detection information and a user state category classification model.

Optionally, the foregoing fatigue category classification model may be obtained through training by using a machine learning method. For example, training may be performed by using a method of a support vector machine (support vector machine, SVM) and a convolutional neural network (convolutional neural network, CNN). By using the training method of the neural network, the fatigue category of the user is determined based on the detected bioelectric signal and a training model, so that the fatigue state of the user is determined more accurately.

With reference to the second aspect, in some implementations of the second aspect, the target device may further update the state category classification model based on fourth detection information from the intelligent control apparatus. When the duration of the detection and heating material working in the heating mode is greater than or equal to a first preset value, the intelligent control apparatus obtains third detection information of the user, and sends, to the target device, the fourth detection information obtained based on the third detection information, so that the target device can update the user state category classification model based on the fourth detection information.

Updating the state category classification model can make the model more accurate. Therefore, when the model is subsequently used to determine the state category of the user, a more accurate determining result can be obtained.

According to a third aspect, an intelligent control apparatus is provided. The apparatus includes a control circuit and a detection and heating circuit. The detection and heating circuit controls, according to an instruction of the control circuit, switching of a working mode of a detection and heating material, where the working mode of the detection and heating material includes a detection mode and a heating mode. In the detection mode, the detection and heating circuit obtains, by using the detection and heating material, first detection information corresponding to a first bioelectric signal. Based on the first detection information, the control circuit instructs the detection and heating circuit to switch the working mode of the detection and heating material to the heating mode, and instructs the detection and heating circuit to control heating duration and/or a heating temperature of the detection and heating material working in the heating mode.

The apparatus may be applied to the field of artificial intelligence. Control by the control circuit enables the detection and heating material to have both detection and heating functions, and intelligent perception of a state of a user is implemented through bioelectric signal detection. In the technical solution of this application, the control circuit and the detection and heating circuit control the detection and heating material to detect a bioelectric signal, and perform heating intervention on the user based on the detected bioelectric signal, thereby implementing a function of intelligently improving the state of the user. In addition, the detection and heating material has two functions: bioelectric signal detection and heating. Therefore, when the intelligent control apparatus is applied to an intelligent wearable device, a material at a same position in the intelligent wearable device is enabled to have both a conventional conducting function and a function of a conventional heating intervention apparatus. On one hand, complexity of the device is reduced. On the other hand, for a position at which an abnormality is detected, intervention can be performed at the position to improve the state of the user at the position more accurately.

Optionally, the detection and heating circuit may use a same structure as the detection and heating circuit in the first aspect, and may perform a same operation or implement a same function as the detection and heating circuit in the first aspect. Details are not described herein again.

Optionally, the control circuit may include a same structure as the control circuit in the first aspect, and/or may perform a same operation as the control circuit in the first aspect, to implement a same function. Details are not described herein again. It should be noted that, in addition to operations that can be performed by the control circuit in the first aspect, the control circuit in the third aspect may further perform operations such as determining a state category of the user by the target device in the second aspect. In other words, the intelligent control apparatus in the third aspect can implement all functions of the intelligent control apparatus in the first aspect, and can also implement all functions of the intelligent system in the second aspect. However, it should be understood that, because all execution modules or units in the third aspect are in the same apparatus, interaction similar to communication between the intelligent control apparatus and the target device in the first aspect does not need to be performed. Therefore, the transceiver circuit and the transceiver in the first aspect and the second aspect may be omitted. However, adding a transceiver circuit or the like in the third aspect does not affect function implementation of the apparatus.

Optionally, the control circuit may include a mode control module, a processing module, an analysis module, and a policy module. It should be understood that the foregoing division is division performed based on functional logic, and another division manner may also exist. It should also be understood that each of the foregoing modules may be implemented in a form of hardware such as a circuit, or may be implemented in a form of a combination of hardware and software. Details are not described herein again.

Optionally, the mode control module may have a same function as the control circuit in the first aspect or the second aspect. In other words, the mode control module may be configured to control the working mode of the detection and heating material, and instruct the detection and heating circuit to adjust a parameter in the working mode of the detection and heating material. Optionally, the processing module may be configured to obtain and process a bioelectric signal or a processed signal of a bioelectric signal from the detection and heating circuit in the detection mode.

In an implementation, the detection and heating circuit obtains the first bioelectric signal of the user, processes the first bioelectric signal, and sends the processed first bioelectric signal (that is, the first detection information) to the processing module. The processing module obtains the processed first bioelectric signal, decomposes the processed first bioelectric signal to obtain a decomposed signal of the first bioelectric signal, and then performs feature extraction on the decomposed signal to obtain a feature signal of the decomposed signal. It should be noted that the foregoing process of signal decomposition and signal feature extraction may alternatively be performed by the detection and heating circuit.

Optionally, the analysis module may perform operations such as determining the state category of the user by the target device, training the state category classification model, and updating the user state category classification model in the first aspect and the second aspect. Optionally, the policy module may perform the process of determining the corresponding improvement solution by the target device based on the state category of the user in the first aspect and the second aspect.

With reference to the third aspect, in some implementations of the third aspect, the detection and heating circuit may be configured to: when the detection and heating material is controlled to work in the detection mode, block an electrical signal that is applied to the detection and heating material and used to control heating of the detection and heating material; or when the detection and heating material works in the heating mode, control the heating temperature of the detection and heating material by controlling an electrical signal applied to the detection and heating material.

With reference to the third aspect, in some implementations of the third aspect, the control circuit may be configured to determine the state category of the user based on the first detection information, and determine an improvement solution corresponding to the state category, where the improvement solution includes the duration and/or the heating temperature of the detection and heating material working in the heating mode; and the control circuit instructs the detection and heating circuit to switch the working mode of the detection and heating material to the heating mode, and instructs the detection and heating circuit to perform the improvement solution. The state category is used to indicate the state of the user, and the state of the user includes at least one of a fatigue state, an anxiety state, a sleep state, a tension state, and a depression state.

Optionally, the intelligent control apparatus may further include a vibration massage apparatus. In this case, when determining the improvement solution, the intelligent control apparatus not only indicates the heating duration and/or the heating temperature of the detection and heating material, but also indicates vibration intervention duration and/or vibration strength of the vibration massage apparatus, or the like.

With reference to the third aspect, in some implementations of the third aspect, the control circuit may be configured to decompose the first detection information to obtain a decomposed signal of the first detection information, perform feature extraction on the decomposed signal to obtain a feature signal of the decomposed signal, and determine the state category of the user based on the feature signal and the state category classification model.

With reference to the third aspect, in some implementations of the third aspect, the control circuit may be further configured to instruct the detection and heating circuit to switch the working mode of the detection and heating material to the detection mode when the duration of the detection and heating material working in the heating mode is greater than or equal to a first preset value; and the detection and heating circuit obtains, by using the detection and heating material, second detection information corresponding to a second bioelectric signal, so that the control circuit can update the state category classification model based on the second detection information.

It should be noted that, in actual application, the intelligent control apparatus provided in this embodiment of this application may further set an initial mode or a power-on mode. For example, the power-on mode may be set to the detection mode. When the user is using the intelligent control apparatus, detection is performed on the user after power-on, and then the heating temperature and/or the heating duration are/is determined based on the detected bioelectric signal. For another example, the initial mode or the power-on mode may alternatively be set to a mode that can be manually selected and switched by the user. For example, a mode selection module may be set, so that the user can autonomously select the initial mode or the power-on mode.

According to a fourth aspect, a control method for an intelligent control apparatus is provided. The method includes: controlling a detection and heating material to work in a detection mode, where a working mode of the detection and heating material includes a heating mode and the detection mode, and the detection and heating material obtains a bioelectric signal of a user when working in the detection mode and performs heating when working in the heating mode; obtaining first detection information obtained based on a first bioelectric signal of the user; sending, to a target device, second detection information obtained based on the first detection information; receiving first instruction information sent by the target device and corresponding to the second detection information; and switching the working mode of the detection and heating material to the heating mode according to the first instruction information, and controlling duration and/or a heating temperature of the detection and heating material working in the heating mode.

Optionally, the foregoing process may be performed by using the corresponding apparatus, circuit, module, or the like provided in the first aspect or the second aspect. In other words, the control method provided in the fourth aspect may be used to control the intelligent control apparatus in the first aspect or the intelligent system in the second aspect.

When the foregoing control method is applied to the apparatus in the first aspect or the system in the second aspect, a corresponding process may be performed as follows: A control circuit instructs a detection and heating circuit to set the working mode of the detection and heating material to the detection mode; in the detection mode, the detection and heating circuit obtains the first bioelectric signal of the user by using the detection and heating material, and processes the first bioelectric signal to obtain the first detection information corresponding to the first bioelectric signal; a transceiver circuit sends, to the target device, the second detection information corresponding to the first detection information; the target device analyzes the second detection information, determines the first instruction information corresponding to the second detection information, and sends the first instruction information to the transceiver circuit; the transceiver circuit receives the first instruction information, and sends the first instruction information to the control circuit; and the control circuit instructs, according to the first instruction information, the detection and heating circuit to switch the working mode of the detection and heating material to the heating mode, and controls the heating duration and/or the heating temperature of the detection and heating material in the heating mode.

It should be noted that the second detection information may be the first detection information itself, or may be a decomposed signal of the first detection information, or may be a feature signal of a decomposed signal of the first detection information.

With reference to the fourth aspect, in some implementations of the fourth aspect, when determining the first instruction information corresponding to the second detection information, the target device may determine a state category of the user based on the second detection information, and then determine the first instruction information based on the state category.

Optionally, the target device may further determine an improvement solution based on the state category, and then generate indication information used to indicate the solution. For example, the target device determines, by analyzing the second detection information, that a fatigue category of the user in the time period is "category 1", and selects, based on a mapping relationship, a mitigation solution in which "the heating temperature is 38°C, massage strength is weak, and operation duration of both heating and massage is 10 minutes"; and accordingly, the target device generates the first instruction information used to indicate the foregoing mitigation solution.

With reference to the fourth aspect, in some implementations of the fourth aspect, when determining the state category of the user based on the second detection information, the target device may determine the state category based on the second detection information and a user state category classification model.

Optionally, the following process may be used to obtain the user state category classification model.

Step 1: The control circuit instructs the detection and heating circuit to set the working mode of the detection and heating material to the detection mode.

Step 2: In the detection mode, the detection and heating circuit obtains a third bioelectric signal of the user by using the detection and heating material, and processes the obtained third bioelectric signal to obtain fifth detection information corresponding to the third bioelectric signal.

Step 3: The transceiver circuit sends, to the target device, sixth detection information corresponding to the fifth detection information, where the sixth detection information may be the fifth detection information itself, or may be a decomposed signal of the fifth detection information, or may be a feature signal of a decomposed signal of the fifth detection information.

Step 5: The target device receives the sixth detection information, and trains the user state category classification model based on the sixth detection information.

Optionally, the machine learning method in the first aspect to the third aspect may be used to train the user state category classification model by using the sixth detection information. The sixth detection information may be a processed third bioelectric signal, a decomposed signal of the third bioelectric signal, or a feature signal of a decomposed signal of the third bioelectric signal.

It should be noted that the third detection information and the fourth detection information may also be obtained by using related methods in the first aspect to the third aspect, and the user state category classification model may be updated based on the fourth detection information.

According to a fifth aspect, a control method for an intelligent control apparatus is provided. The method includes: controlling a detection and heating material to work in a detection mode, where a working mode of the detection and heating material includes a heating mode and the detection mode, and the detection and heating material obtains a bioelectric signal of a user when working in the detection mode and performs heating when working in the heating mode; obtaining first detection information corresponding to a first bioelectric signal; switching the working mode of the detection and heating material to the heating mode based on the first detection information, and controlling duration and/or a heating temperature of the detection and heating material working in the heating mode.

Optionally, the foregoing process may be performed by using the corresponding apparatus, circuit, module, or the like provided in the third aspect. In other words, the control method provided in the fifth aspect may be used to control the intelligent control apparatus in the third aspect.

Optionally, a control circuit may include a mode control module, a processing module, an analysis module, and a policy module. It should be understood that the foregoing division is division performed based on functional logic, and another division manner may also exist. It should also be understood that each of the foregoing modules may be implemented in a form of hardware such as a circuit, or may be implemented in a form of a combination of hardware and software. Details are not described herein again.

Based on composition of the control circuit, the control method may be performed by using the following process.

Step 1: The control module instructs a detection and heating circuit to set the working mode of the detection and heating material to the detection mode.

Step 2: In the detection mode, the detection and heating circuit obtains the first bioelectric signal of the user by using the detection and heating material, and processes the obtained first bioelectric signal to obtain a digital first bioelectric signal.

Step 3: The detection and heating circuit transmits the digital first bioelectric signal to the processing module by using a wire or a data sending mode.

Step 4: The processing module performs processing such as decomposition and feature extraction on the digital first bioelectric signal, and sends an obtained feature signal to the analysis module.

Step 5: The analysis module receives the feature signal, determines a state category of the user based on the feature signal, and sends the state category of the user to the policy module.

Step 6: The policy module obtains the state category, determines a corresponding improvement solution based on the state category, and sends the improvement solution to the mode control module.

Step 7: The mode control module converts the improvement solution into an instruction that can indicate the solution, instructs the detection and heating circuit to switch the working mode of the detection and heating material, so that the detection and heating material works in the heating mode instead, and instructs the detection and heating circuit to control the heating duration and/or the heating temperature of the detection and heating material, so that the detection and heating circuit performs the improvement solution.

Step 8: The detection and heating circuit switches the working mode of the detection and heating material to the heating mode according to the instruction of the mode control module.

Step 9: In the heating mode, the detection and heating circuit controls the heating duration and/or the heating temperature of the detection and heating material according to the instruction of the mode control module, to perform the improvement solution.

With reference to the fifth aspect, in some implementations of the fifth aspect, when the detection and heating material works in the detection mode, an electrical signal that is applied to the detection and heating material and used to control heating of the detection and heating material may be blocked; or when the detection and heating material works in the heating mode, the heating temperature of the detection and heating material is controlled by controlling an electrical signal applied to the detection and heating material.

With reference to the fifth aspect, in some implementations of the fifth aspect, the state category of the user may be determined based on the first detection information, and the improvement solution corresponding to the state category is determined, where the improvement solution includes the duration and/or the heating temperature of the detection and heating material working in the heating mode; and the control circuit instructs the detection and heating circuit to switch the working mode of the detection and heating material to the heating mode, and instructs the detection and heating circuit to perform the improvement solution. The state category is used to indicate a state of the user, and the state of the user includes at least one of a fatigue state, an anxiety state, a sleep state, a tension state, and a depression state.

With reference to the fifth aspect, in some implementations of the fifth aspect, the first detection information may be decomposed to obtain a decomposed signal of the first detection information, feature extraction is performed on the decomposed signal to obtain a feature signal of the decomposed signal, and the state category of the user is determined based on the feature signal and a state category classification model.

With reference to the fifth aspect, in some implementations of the fifth aspect, when the duration of the detection and heating material working in the heating mode is greater than or equal to a first preset value, the detection and heating circuit may be further instructed to switch the working mode of the detection and heating material to the detection mode; and the detection and heating circuit obtains, by using the detection and heating material, second detection information corresponding to a second bioelectric signal, so that the control circuit can update the state category classification model based on the second detection information.

According to a sixth aspect, an intelligent wearable device is provided. The intelligent wearable device includes any intelligent control apparatus in the first aspect or the third aspect.

Optionally, the intelligent wearable device may further include a wearable device body. Optionally, a detection and heating material of the intelligent control apparatus may be a component of the wearable device body.

With reference to the sixth aspect, in some implementations of the sixth aspect, the intelligent wearable device is an intelligent eye mask, and the intelligent eye mask includes an eye mask body and an intelligent control apparatus. The intelligent control apparatus may be any intelligent control apparatus in the first aspect or the third aspect.

It should be understood that the intelligent control apparatus may be disposed in an interlayer of the eye mask body, or a detection and heating material of the intelligent control apparatus may be used as a part of the eye mask body. For example, the detection and heating material is directly woven on an outer surface of the eye mask body in a weaving manner.

It should also be understood that the intelligent control apparatus provided in this embodiment of this application may be further applied to another wearable device.

Optionally, any intelligent control apparatus in the first aspect or the third aspect may be applied to a virtual reality (virtual reality, VR) device, for example, VR glasses, to intelligently mitigate user fatigue, for example, eye fatigue.

For example, the intelligent control apparatus in the first aspect and an intelligent analysis apparatus may be disposed in the VR glasses. The intelligent control apparatus includes a detection and heating circuit, a transceiver circuit, and a control circuit. The control circuit is configured to control a detection and heating material to detect a bioelectric signal or perform heating. The intelligent analysis apparatus is configured to determine a state category of a user based on received detection information (the bioelectric signal, a feature signal of the bioelectric signal, or the like), and/or configured to determine an improvement solution based on a fatigue category. This is equivalent to being configured to perform an operation of the foregoing target device. When working, the detection and heating material detects the bioelectric signal of the user, and sends, to the intelligent analysis apparatus by using the transceiver circuit, the detection information corresponding to the bioelectric signal. The intelligent analysis apparatus determines the fatigue category of the user based on the signal feature of the received bioelectric signal, and sends indication information about the fatigue category of the user or the improvement solution to the control circuit by using the transceiver circuit, so that the control circuit can control heating duration and/or a heating temperature of the detection and heating material accordingly.

For another example, any intelligent system in the second aspect may be further disposed in the VR glasses.

For another example, any intelligent control apparatus in the third aspect may be further disposed in the VR glasses. The intelligent control apparatus includes a detection and heating circuit and a control circuit. The detection and heating circuit includes a detection and heating material for detecting a bioelectric signal and performing certain processing on the bioelectric signal. The control circuit is configured to perform an operation such as determining an improvement solution based on the bioelectric signal. For a specific process, refer to the related description above. For brevity, details are not described again.

Further, to make services of the VR glasses more personalized, vibration massage patches may be further disposed near eye acupoints, massage strength and/or duration of the vibration massage patches are/is controlled based on the fatigue category of the user, and different heating duration and/or different heating temperatures may be set for different positions.

Optionally, any intelligent control apparatus in the first aspect may be further applied to a smart belt for mitigating lower back pain, and the heating duration and/or the heating temperature of the detection and heating material may be controlled when an abnormality is determined based on the bioelectric signal detected by the detection and heating material, to mitigate the lower back pain or fatigue.

Further, to make services of the wearable device more personalized, intervention in a manner of heating and massage or the like can be further focused on different abnormal positions. For example, in the smart belt for mitigating low back pain, if the detection and heating material detects an abnormal bioelectric signal at a position of a lumbar vertebra, auxiliary vibration massage may be performed at the position during heating.

In addition, any intelligent control apparatus in the first aspect or the third aspect, and any intelligent system in the second aspect may be further applied to another wearable device such as a head mounted massage device. Details are not described herein again.

In the technical solution of this application, any intelligent control apparatus provided in the first aspect or the third aspect or any intelligent system provided in the second aspect is applied to a wearable device such as an intelligent eye mask, a VR device, or a smart belt to implement a function of intelligently improving a state of the user, and the detection and heating material is applied to reduce structural complexity of the wearable device and improve wear comfort of the user. In addition, in the foregoing example, intervention in a manner of heating and massage or the like can be further focused on different abnormal positions, so that the services of the wearable device are more personalized.

According to a seventh aspect, this application provides a computer-readable storage medium, where the computer-readable storage medium stores computer instructions, and when the computer instructions are run on a computer, the computer is enabled to perform the method in any one of the fourth aspect or the possible implementations of the fourth aspect, and/or the computer is enabled to perform the method in any one of the fifth aspect or the possible implementations of the fifth aspect.

According to an eighth aspect, this application provides a computer program product, where the computer program product includes computer program code, and when the computer program code is run on a computer, the computer is enabled to perform the method in any one of the fourth aspect or the possible implementations of the fourth aspect, and/or the computer is enabled to perform the method in any one of the fifth aspect or the possible implementations of the fifth aspect.

**BRIEF DESCRIPTION OF DRAWINGS**

FIG. 1 is a schematic diagram of an intelligent system according to an embodiment of this application;
FIG. 2 is a schematic diagram of a structure of a detection and heating material and a connected circuit according to an embodiment of this application;
FIG. 3 is a schematic diagram of a structure of a detection and heating material and a connected circuit according to an embodiment of this application;
FIG. 4 is a schematic diagram of a structure of a detection circuit according to an embodiment of this application;
FIG. 5 is a schematic diagram of a structure of a convolutional neural network according to an embodiment of this application;
FIG. 6 is a schematic flowchart of a control method for an intelligent control apparatus according to an embodiment of this application;
FIG. 7 is a schematic flowchart of a method for obtaining a user state category classification model according to an embodiment of this application;
FIG. 8 is a schematic flowchart of a method for updating a user state category classification model according to an embodiment of this application;
FIG. 9 is a schematic diagram of an intelligent control apparatus according to an embodiment of this application;
FIG. 10 is a schematic diagram of a hardware structure of an intelligent control apparatus according to an embodiment of this application;
FIG. 11 is a schematic diagram of an intelligent control apparatus according to an embodiment of this application;
FIG. 12 is a schematic flowchart of a control method for an intelligent control apparatus according to an embodiment of this application;
FIG. 13 is a schematic flowchart of a method for obtaining a user state category classification model according to an embodiment of this application;
FIG. 14 is a schematic flowchart of a method for updating a user state category classification model according to an embodiment of this application;
FIG. 15 is a schematic diagram of a hardware structure of an intelligent control apparatus according to an embodiment of this application; and

FIG. 16 is a schematic diagram of an intelligent eye mask according to an embodiment of this application.

**DESCRIPTION OF EMBODIMENTS**

The following describes the technical solutions of this application with reference to the accompanying drawings.
For ease of understanding the technical solutions of this application, technical terms and concepts in this application are described herein.

(1) Bioelectric signal

Bioelectricity refers to a regular electrical phenomenon closely related to a state of life and generated regardless of whether living cells or tissues (a human body or animal tissue) are in a static state or an active state.
Bioelectric signals include resting potentials and action potentials, and their essence is a transmembrane flow of ions. More common bioelectric signals are electromyography signals, electroencephalogram signals, electrocardiosignals, electro-oculogram signals, and the like. These bioelectric signals that can be obtained from body surface measurements are also referred to as body surface bioelectric signals. Because there are certain differences in frequency bands, amplitudes, waveforms, and the like of bioelectric signals such as electromyography signals, electroencephalogram signals, electrocardiosignals, and electro-oculogram signals, one or more bioelectric signals may be obtained by processing bioelectric signals collected by an electrode. In the conventional technology, these bioelectric signals are often recorded as an electromyogram, an electroencephalogram, an electrocardiogram, and the like, to provide help for relevant professionals in scientific research and medical diagnosis.

(2) Electroencephalogram signal

An electroencephalogram (electroencephalogram, EEG) signal, also referred to as a brain wave signal, is an external reflection of a brain activity. Different brain activities are represented by electroencephalogram signals with different features. Research shows that time-space-frequency domain analysis of these electroencephalogram patterns is helpful to reversely analyze intentional human activities, and this provides a theoretical basis for application of electroencephalogram signals.
A scalp electroencephalogram signal is a bioelectric signal. After an electric field formed by potential changes of 86 billion neurons in the brain is conducted by a volume conductor including a cortex, a skull, meninges, and a scalp, a potential distribution is generated on the scalp. Electroencephalogram signals can be obtained by recording the potential distribution of these changes by using a particular device.
The electroencephalogram signals may be further classified into two types: spontaneous (spontaneous) electroencephalogram and evoked potential (evoked potential, EP) electroencephalogram. The spontaneous electroencephalogram is a spontaneous potential change generated by nerve cells in the brain in absence of specific external stimulation. The evoked potential electroencephalogram is a brain potential change caused by different types of stimulation such as sound, light, and electricity on nerve cells in the brain. By detecting human electroencephalogram signals, a state of the human body can be obtained. Usually, human beings mainly generate several electroencephalogram signals: $\delta$, $\theta$, $\alpha$, $\beta$, and $\gamma$.
$\delta$ wave: Its frequency is between 1 Hz and 3 Hz. It is more obvious in a parietal lobe and a pituitary gland, and is more obvious in infancy or when mental development is immature. It is a slow wave. In a normal case, the $\delta$ wave exists only in a state in which there is an extreme lack of oxygen, a deep sleep state, a state in which there is a cerebral disease, or the like.
$\theta$ wave: Its frequency is between 4 Hz and 7 Hz. It is a slow wave. It mainly appears in an occipital region and a parietal occipital region, and is symmetric on left and right sides. Generally, it can be detected when a person is sleepy or in light sleep. In addition, it is generally related to a psychological state of the person. Usually, when the person feels depressed, frustrated, or sleepy, a central nervous system is in a depressed state, and the wave appears.
$\alpha$ wave: Its frequency is between 8 Hz and 12 Hz. It mainly appears in the parietal occipital region, and the two sides are basically synchronized. It is a basic rhythm of an EEG of a normal person. When an individual is thinking or in a rest state, the wave is relatively obvious, and when the individual undertakes a targeted activity, opens eyes, or receives other stimuli, the wave disappears, and a $\beta$ wave appears instead.
$\beta$ wave: Its frequency is between 12.5 Hz and 28 Hz. It mainly appears in frontal and central regions. The frequency of this wave significantly represents an excitement level of a cerebral cortex. This wave appears when the individual is in an awake state and an early drowsiness period.
$\gamma$ wave: Its frequency is between 25 Hz and 100 Hz. It mainly appears in the frontal and central regions. The frequency of this wave significantly represents the excitement level of the cerebral cortex. This wave appears when the individual is in the awake state and the early drowsiness period.

In this application, to obtain a mental state such as a fatigue state of a person by detecting a spontaneous electroencephalogram signal, spontaneous electroencephalogram signals may be classified into different brain wave types based on different frequency ranges, and a correspondence between the different brain wave types and human body states is established, as shown in Table 1.

**Table 1**

| Brain wave type | | Frequency (Hz) | State of the human body |
|---|---|---|---|
| Delta ($\delta$) | | 1 to 3 | Deep sleep without dreaming |
| Theta ($\theta$) | | 4 to 7 | Adult emotional stress, such as disappointed or frustrated |
| Alpha ($\alpha$) | | 8 to 12 | Relaxed and calm, with eyes closed, but awake |
| Beta ($\beta$) | L | 12.5 to 16 | Relaxed but concentrated |
| | M | 16.5 to 20 | Thinking, processing, and receiving external messages |
| | H | 20.5 to 28 | Excited and anxious |
| Gamma ($\gamma$) | | 25 to 100 | Enhancing awareness and happiness, reducing stress, and deep thought |

In an example, a manner of obtaining brain waves may be separately obtaining brain waves at positions such as the occipital region, the parietal occipital region, the parietal lobe, the pituitary gland, and the frontal. For example, a user wears a hood on the head, where a plurality of brain wave capture modules are disposed in the hood, and the plurality of brain wave capture modules correspond to the positions such as the occipital region, the parietal occipital region, the parietal lobe, the pituitary gland, and the frontal, to measure the brain waves at the positions. Therefore, $\delta$, $\theta$, $\alpha$, $\beta$, and $\gamma$ brain waves of the user can be obtained.

(3) Electromyography signal

An electromyography (electromyography, EMG) signal is a superposition of action potentials of motion units in a plurality of muscle fibers in time and space. A surface electromyography signal is an electrical signal associated with muscle contraction. An electromyography signal detection method is an important method for non-invasively detecting a muscle activity on a body surface. The electromyography signal in the embodiments of this application mainly refers to a surface electromyography signal. For example, the electromyography signal may include an electromyography signal of a facial action such as frowning or tooth gritting by the user.

(4) Electro-oculogram signal

An electro-oculogram (electro-oculogram, EOG) signal is an electrical signal that can reflect an eye state. For example, the electro-oculogram signal may include an electro-oculogram signal of a horizontal eye movement, an eye blink frequency, or eye closing duration of the user.

(5) Blind signal separation and independent component analysis

Blind source separation (blind source separation, BSS), also referred to as blind signal separation, refers to a process of separating various source signals from a mixed signal (for example, the foregoing bioelectric signal) when a theoretical model of the signal and the source signals cannot be accurately obtained. Except statistical independence, there is no prior knowledge about these source signals. Blind source signal separation is a powerful signal processing method, and has been widely applied in fields such as biomedical signal processing, array signal processing, speech signal recognition, image processing, and mobile communications.

Independent component analysis (independent component analysis, ICA) is a common blind signal separation method. Basic ICA is a technology for separating source signals from a linearly mixed signal of a plurality of source signals, and has become a powerful tool for array signal processing and data analysis.

(6) Support vector machine

The support vector machine (support vector machine, SVM) is a supervised learning model and related learning algorithm for analyzing data in classification and regression analysis. The SVM is mainly used to analyze a linearly separable case. For a linearly inseparable case, a linearly inseparable sample of a low-dimensional input space is transformed into a high-dimensional eigenspace by using a nonlinear mapping algorithm, so that the sample is linearly separable. Therefore, it is possible to use a linear algorithm in the high-dimensional eigenspace to linearly analyze a nonlinear feature of the sample. Based on the structural risk minimization principle, an optimal hyperplane is constructed in the eigenspace, so that the learner is optimized globally, and the expectation of the whole sample space satisfies an upper boundary with a certain probability.

In the embodiments of this application, the SVM is used to classify the mental state of the person based on the bioelectric signal such as the electroencephalogram signal, the electromyography signal, or the electro-oculogram signal.

(7) Convolutional neural network

The convolutional neural network (convolutional neural network, CNN) is a deep neural network with a convolutional structure. The convolutional neural network includes a feature extractor including a convolutional layer and a sub-sampling layer. The feature extractor may be considered as a filter. The convolutional layer is a neuron layer that performs convolution processing on an input signal that is in the convolutional neural network. In the convolutional layer of the convolutional neural network, one neuron may be connected to only a part of neurons in a neighboring layer. A convolutional layer generally includes several feature planes, and each feature plane may include some neurons arranged in a rectangle. Neurons of a same feature plane share a weight, and the shared weight herein is a convolution kernel. Sharing the weight may be understood as that a manner of extracting image information is unrelated to a position. The convolution kernel may be initialized in a form of a matrix of a random size. In a training process of the convolutional neural network, an appropriate weight may be obtained for the convolution kernel through learning. In addition, sharing the weight is advantageous because connections between layers of the convolutional neural network are reduced, and a risk of overfitting is reduced.

(8) Loss function

In a process of training a deep neural network, because it is expected that an output of the deep neural network is as close as possible to a value that is actually expected to be predicted, a predicted value of a current network and a target value that is actually expected may be compared, and then, a weight vector of each layer of the neural network is updated based on a difference between the two (certainly, there is usually an initialization process before the first update, that is, a parameter is preconfigured for each layer in the deep neural network). For example, if the predicted value of the network is higher, the weight vector is adjusted to obtain a lower predicted value. The weight vector is continuously adjusted until the deep neural network can predict the target value that is actually expected or a value that is very close to the target value that is actually expected. Therefore, "how to obtain, through comparison, a difference between the predicted value and the target value" needs to be predefined. This is a loss function (loss function) or an objective function (objective function). The loss function and the objective function are important equations used to measure the difference between the predicted value and the target value. The loss function is used as an example. A higher output value (loss) of the loss function indicates a larger difference. Therefore, training of the deep neural network becomes a process of reducing the loss as much as possible.

(9) Back propagation algorithm

In a training process, a neural network may correct values of parameters in an initial neural network model by using an error back propagation (back propagation, BP) algorithm, so that a reconstruction error loss of the neural network model becomes increasingly smaller. Specifically, an input signal is forward transferred until an error loss occurs during output, and the parameters in the initial neural network model are updated based on back propagation error loss information, so that the error loss is reduced. The back propagation algorithm is a back propagation motion mainly dependent on the error loss, and aims to obtain parameters of an optimal neural network model, for example, a weight matrix.

In the embodiments of this application, a material having a conductivity and/or heat conductivity characteristic is applied to an intelligent wearable device by using the two conductivity and heat conductivity characteristics of the material, and functions of both the two characteristics can be implemented by using a circuit design, to perform intelligent intervention on the mental state such as fatigue of the user.

It should be understood that the intelligent wearable device in the embodiments of this application may be in an eye mask form, a head mask form, or a face mask form. When relating to a non-head bioelectric signal that can be obtained, such as an electrocardiosignal, the intelligent wearable device may alternatively be in a fingerstall form, a wristband form, or the like. Details are not described herein.

Generally, in the intelligent wearable device, if a bioelectric signal of the human body needs to be captured, an electrode is used. For wear comfort, a flexible electrode, such as a fabric electrode, a foam electrode, or a rubber electrode, is generally used. For the fabric electrode, a fabric having conductivity may be woven into a base material of the intelligent wearable device, and a conducting wire is connected to another circuit module by using a lead or a conducting braided wire, to form the fabric electrode.

If the intelligent wearable device needs to perform intervention on the human body, an intervention apparatus is also needed. For example, the intervention apparatus may be a heating element or apparatus, a vibration massage element or apparatus, an audio playback apparatus, or a video playback apparatus, and the intelligent wearable device is configured to intervene in the mental state such as fatigue of the user. In the embodiments of this application, a heating intervention apparatus and a combination of the heating intervention apparatus and the vibration massage apparatus are mainly used as examples for description.

FIG. 1 is a schematic diagram of an intelligent system according to an embodiment of this application. For example, the intelligent system may be configured to intelligently perceive and improve a state of a user. As shown in FIG. 1, the intelligent system includes an intelligent control apparatus 100 and a target device 200. The intelligent control apparatus 100 may include a detection and heating circuit 110, a control circuit 120, and a transceiver circuit 130. The target device 200 may include a transceiver 210 and a processor 220. The following separately describes various elements in FIG. 1 and functions of the elements.

The detection and heating circuit 110 includes a detection and heating material. The detection and heating material has two working modes: a detection mode and a heating mode. The detection and heating material is used to obtain a bioelectric signal of the user in the detection mode, and used for heating in the heating mode.

Optionally, the detection and heating material may be used to obtain bioelectric signals of different parts of a body of the user. For example, the bioelectric signals include an electroencephalogram signal of a head, an electro-oculogram signal of an eye, and an electromyography signal of a face, and may further include an electrocardiosignal, and an electrical signal of a limb part. In other words, the bioelectric signal in this embodiment of this application may include one or more of the foregoing bioelectric signals. For ease of description, in this embodiment of this application, it is assumed that the foregoing electroencephalogram signal, electro-oculogram signal, and electromyography signal are included. However, it should be understood that this is only an example for description and does not constitute a limitation on the bioelectric signals.

It should be understood that when the detection and heating material works in the detection mode, the detection and heating material has a same function as a conventional electrode, that is, used to detect a bioelectric signal; or when the detection and heating material works in the heating mode, the detection and heating material also has a same function as a conventional heating intervention apparatus, that is, used to perform intervention on the user through heating, thereby improving a mental state of the user.

In this embodiment of this application, the detection and heating material has two functions: bioelectric signal detection and heating, which is equivalent to having both a function of a conventional electrode and a function of a conventional intervention apparatus. When the detection and heating material or the intelligent control apparatus is applied to an intelligent wearable device, a material at a same position in the intelligent wearable device is enabled to have both the function of the conventional electrode and the function of the conventional heating intervention apparatus. On one hand, complexity of the device is reduced. On the other hand, for a position at which an abnormality is detected, intervention can be performed at the position to improve the state of the user at the position more accurately.

The conventional intervention apparatus may include various intervention apparatuses for heating, massage, music playback, video playback, and the like that can intervene in a physiological state and/or the mental state of the user. For example, a state such as fatigue or soreness may be mitigated through heating massage or vibration massage, and a brain of the user may be relaxed through listening to music or viewing a video, thereby mitigating tension of the user.

When detecting a bioelectric signal, the conventional electrode may obtain a bioelectric signal at a detection electrode by measuring a potential difference between the detection electrode and a reference electrode.

Therefore, in an implementation, when the detection and heating material works in the detection mode, the bioelectric signal of the user may be obtained by measuring a potential difference between a to-be- measured position and a reference position.

Optionally, when the detection and heating material works in the heating mode, an electrical signal may be applied to the detection and heating material to heat the detection and heating material, and a voltage and/or current of the applied electrical signal are/is controlled to control a heating temperature of the detection and heating material.

In an example, in consideration of comfort, when the intelligent control apparatus 100 is used for the intelligent wearable device, the detection and heating material may be woven in a base material of the intelligent wearable device to make a fabric that has both detection and heating functions.

Optionally, the detection and heating material may be made of one or more of materials such as silver plating, silver chloride plating, and graphene fiber.

In an example, the detection and heating material is machined from a combination of a conducting material and a heat conducting material. The conducting material is used to obtain the bioelectric signal of the user, and the heat conducting material is used for heating. It should be understood that, in this example, the conducting material may be a material having only conductivity, and the heat conducting material may be a material having only heat conductivity. For example, the conducting material may be an inorganic water-absorbing and water-retaining material, an organic material of a hydrophilic functional group, an alloy material, or a metal material, and the heat conducting material may be a heat conducting silica gel, a heat conducting silicone, a carbon-based material, an alloy material, or a metal material. These material components undergo fiber processing, micro-nano processing, and the like. For example, the material is attached to a fiber surface or a foam tissue surface, or the material is machined onto another substrate through micro-nano processing. In this way, the detection and heating material is machined from the conducting material and the heat conducting material.

For further example, the conducting material and the heat conducting material may be woven into the detection and heating material in a weaving manner. It should be understood that the two materials may also be made into the detection and heating material through combined machining in other manners.

FIG. 2 is a schematic diagram of a structure of a detection and heating material and a connected circuit according to an embodiment of this application. As shown in FIG. 2, a conducting material and a heat conducting material are woven into a detection and heating material, and connected to other modules in a detection and heating circuit 110. The following provides descriptions with reference to FIG. 2.

As shown in FIG. 2, a detection and heating material 310 includes a conducting material 311 and a heat conducting material 312, both of which are woven into the detection and heating material 310 for bioelectric signal detection and/or heating. The conducting material 311 is used to detect a bioelectric signal, and the heat conducting material 312 is used for heating.

Optionally, a cross-weaving manner may be used. However, it should be understood that the weaving manner is not limited in this embodiment of this application. For example, another weaving manner such as front-back weaving may also be used. It should also be understood that the two materials may alternatively be, for example, only stacked and fastened together without being woven.

It should be understood that both the conducting material 311 and the heat conducting material 312 may be made of a mixture of one or more materials. To be specific, the conducting material 311 may be made of one or more materials having conductivity, and the heat conducting material 312 may be made of one or more materials having heat conductivity. It should also be understood that the conducting material may be a material having only conductivity, or may be a material having both conductivity and heat conductivity. Similarly, the heat conducting material may be a material having only heat conductivity, or may be a material having both conductivity and heat conductivity.

As shown in FIG. 2, the conducting material 311 is connected to a detection circuit 320 in a detection and heating circuit 300, and the heat conducting material 312 is connected to a heating circuit 330 in the detection and heating circuit 300.

Optionally, wires may be used to connect the conducting material 311 to the detection circuit 320 and connect the heat conducting material 312 to the heating circuit 330.

Optionally, when the detection and heating material 310 works in a detection mode, the conducting material 311 may detect a bioelectric signal of a user, and transmit the bioelectric signal to the detection circuit 320 by using a wire, and the detection circuit 320 may obtain the bioelectric signal and perform corresponding processing. For example, the detection circuit 320 may use a circuit or module having a same function as a detection circuit 400 shown in FIG. 4.

Optionally, when the detection and heating material 310 works in a heating mode, the heating circuit 330 in the detection and heating circuit 300 controls heating of the heat conducting material 312 and controls a heating temperature of the heat conducting material 312.

Optionally, the heating circuit 330 may control whether to perform heating and control the heating temperature by controlling on/off and strength of an electrical signal applied to the heat conducting material 312, where the electrical signal may be a voltage and/or current signal.

It should be noted that the conducting material 311 in the detection and heating material 310 is used only for bioelectric signal detection and not used for heating; and the heat conducting material 312 is used only for heating, and not used for bioelectric signal detection. In other words, even if the conducting material 311 is a material having both conductivity and heat conductivity, the conducting material 311 is only enabled to implement a conducting function and used for bioelectric signal detection; and even if the heat conducting material 312 is a material having both conductivity and heat conductivity, the heat conducting material 312 is only enabled to implement a heat conducting function and used for heating.

In FIG. 2, the conducting material 311 and the heat conducting material 312 are disposed at a same position after certain machining, so that the material at the same position has both the conducting function and the heat conducting function. However, in different working modes, only one of the conducting material 311 and the heat conducting material 312 generally works. In other words, only the conducting material 311 works in the detection mode, and only the heat conducting material 312 works in the heating mode. When the detection and heating material 310 shown in FIG. 2 is used for intelligently perceiving a state of the user and intelligently improving the state of the user, after an abnormal bioelectric signal is detected by using a conducting material at a position, heating intervention can be performed by using a heat conducting material at the same position, to improve a body part more accurately and improve user experience.

In another example, through weaving or sticking, the detection and heating material may be made of one or more materials having both conductivity and heat conductivity. For example, a silver plating material may be woven into the detection and heating material, or a silver plating material and a graphene fiber material may be woven into the detection and heating material for both heating and bioelectric signal detection.

Optionally, as shown in FIG. 3, one or more materials having both conductivity and heat conductivity may be woven into a detection and heating material, and the detection and heating material is connected to a detection and heating circuit.

FIG. 3 is a schematic diagram of a structure of a detection and heating material and a connected circuit according to an embodiment of this application. The following provides descriptions with reference to FIG. 3. As shown in FIG. 3, through weaving, a detection and heating material 310' may be made of one or more materials having both conductivity and heat conductivity.

It should be noted that, a difference between the detection and heating material 310' shown in FIG. 3 and the detection and heating material 310 shown in FIG. 2 lies in that the material in the detection and heating material 310' may be used for both bioelectric signal detection and heating.

Optionally, the detection and heating material 310' may use a cross-weaving manner. However, it should be understood that the weaving manner is not limited in this embodiment of this application. For example, a weaving manner such as front-back weaving may be used. It should also be understood that the detection and heating material 310' may be made in other manners than weaving, and details are not described herein again.

As shown in FIG. 3, the detection and heating material 310' is connected to both a detection circuit 320' and a heating circuit 330'. Optionally, the detection and heating material 310' may be connected to both the detection circuit 320' and the heating circuit 330' by using wires.

It should be noted that in the detection and heating material 310', a part of the material may be set not to work. For example, a part of the material may be used only as a fabric and not connected to the circuit; or a part of the material may be connected to only one circuit. For further example, assuming that the detection and heating material 310' is divided into 10 sub-materials in total, that is, the 10 sub-materials may be used for capturing bioelectric signals and/or heating, there may be the following several cases:

Case 1: The 10 sub-materials are all connected to the detection circuit 320' and the heating circuit 330'. In this case, the 10 sub-materials can be used for both bioelectric signal detection and heating.

Case 2: Apart of the 10 sub-materials are connected to two circuits, and the rest are not connected to any circuit. For example, five of the 10 sub-materials are connected to both the detection circuit 320' and the heating circuit 330', and the remaining five sub-materials are not connected to any circuit and are used only as ordinary fabrics.

Case 3: A part of the 10 sub-materials are connected to two circuits, and a part of the 10 sub-materials are connected to one circuit. For example, five sub-materials are connected to both the detection circuit 320' and the heating circuit 330', two sub-materials are connected to only the detection circuit 320', and three sub-materials are connected to only the heating circuit 330'. In this case, seven sub-materials can be used for detection in a detection mode, and eight sub-materials can be used for heating in a heating mode.

Case 4: A part of the 10 sub-materials are connected to two circuits, a part of the 10 sub-materials are connected to one circuit, and a part of the 10 sub-materials are not connected to any circuit. For example, five sub-materials are connected to both the detection circuit 320' and the heating circuit 330', one sub-material is connected to only the detection circuit 320', two sub-materials are connected to only the heating circuit 330', and two sub-materials are not connected to any circuit. In this case, six sub-materials can be used for detection in the detection mode, and seven sub-materials can be used for heating in the heating mode.

In other words, only a part of the material may be enabled to work in two modes.

In one case, when a detection and heating circuit 300' sets the detection and heating material 310' to work in the detection mode, a bioelectric signal of the user may be obtained by using the detection and heating material 310' and transmitted to the detection circuit 320' by using a wire; and the detection circuit 320' may obtain the bioelectric signal and perform corresponding processing to obtain corresponding detection information. Optionally, to avoid adverse impact on the bioelectric signal detection process, an electrical signal applied to the detection and heating material 310' by the heating circuit 330' may be further shielded by the detection circuit 320'.

In another case, when the detection and heating circuit 300' sets the detection and heating material 310' to work in the heating mode, the detection and heating circuit 300' may apply an electrical signal such as a current or a voltage to the detection and heating material 310' by using the heating circuit 330', so that the detection and heating material 310' performs heating, and the detection and heating circuit 300' may control strength of the electrical signal to control a heating temperature of the detection and heating material 310'.

Optionally, the detection and heating circuit 300' may further control heating duration of the detection and heating material 310' by controlling duration of applying the electrical signal to the detection and heating material 310'. For example, a timer may be set. After the detection and heating circuit 300' connects the electrical signal applied to a material 312', timing is started. After a time specified by the timer expires, the electrical signal is disconnected. In this case, the heating duration of the material can be controlled.

Optionally, a switch may be set on the detection circuit 320' and the heating circuit 330', and the electrical signal applied to the detection and heating material 310' can be controlled by controlling on/off of the switch. In other words, in the detection mode, the detection and heating material 310' is connected to the detection circuit 320' (that is, there is an electrical connection), and is disconnected from the heating circuit 330' (that is, there is no electrical connection); or in the heating mode, the detection and heating material 310' is connected to the heating circuit 330' (that is, there is an electrical connection), and is disconnected from the detection circuit 320' (that is, there is no electrical connection).

Optionally, when the detection and heating material 310' works in the detection mode, the electrical signal connected by using the heating circuit 330' may be blocked. To be specific, in the detection mode, the electrical signal applied to the detection and heating material 3 10' by the heating circuit 330' is blocked; or in the heating mode, the electrical signal is applied to the detection and heating material 310' by the heating circuit 330' to heat the detection and heating material 310', and the temperature of the detection and heating material 310' is controlled by controlling the strength of the applied electrical signal. It should be understood that, in this example, the electrical signal applied to the detection and heating material 310' by the heating circuit 330' may not be disconnected in another form such as a switch, but in the detection mode, a blocking circuit is set to block the electrical signal applied to the detection and heating material 310' by the heating circuit 330'.

Optionally, during bioelectric signal detection, a direct current or working frequency suppression module (for example, the module may be a direct current or working frequency suppression module 410 shown in FIG. 4) may be used to block a direct current signal used to control heating, so that no interference is caused to the bioelectric signal detection process. When bioelectric signal detection is not performed, the electrical signal used to control heating of the detection and heating material is applied to heat the detection and heating material, and the temperature of the detection and heating material is controlled by the strength of the applied electrical signal.

Optionally, the detection and heating materials shown in FIG. 2 and FIG. 3 may be further used in combination. In other words, the detection and heating material includes a material part having both conductivity and heat conductivity as shown in FIG. 3, and this material part is connected to both the detection circuit and the heating circuit; and the detection and heating material further includes a material part having only conductivity or having only heat conductivity as shown in FIG. 2, and in this material part, the conducting material is connected to the detection circuit, and the heat conducting material is connected to the heating circuit. It should be noted that regardless of FIG. 2, FIG. 3, or the foregoing example, there may be a material used only as an ordinary fabric and not connected to any circuit.

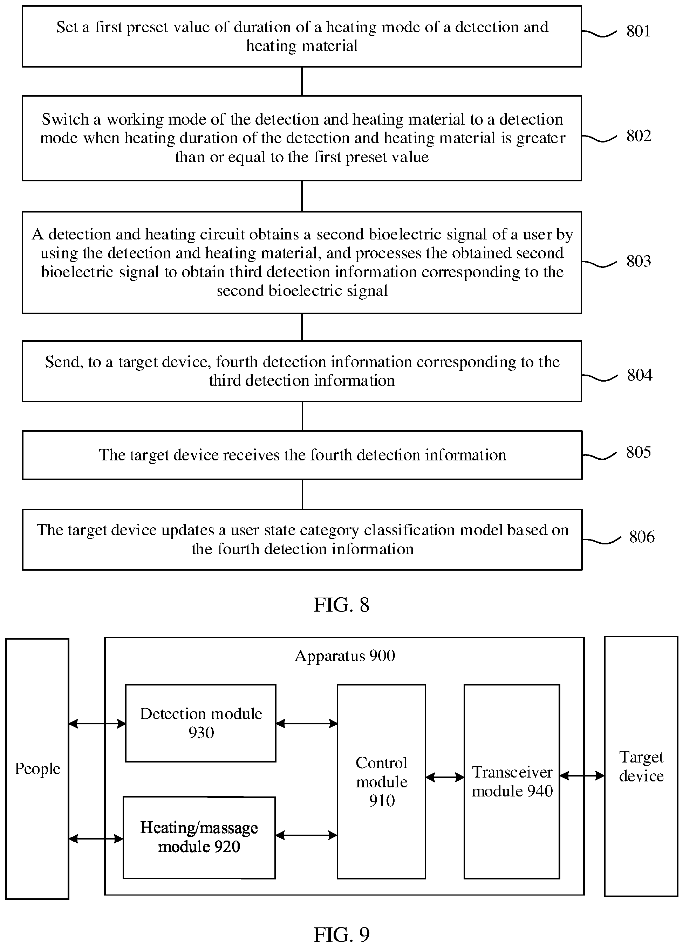

FIG. 4 is a schematic diagram of a structure of a detection circuit according to an embodiment of this application. The detection circuit shown in FIG. 4 may be configured to process a bioelectric signal obtained by a detection and heating material, to obtain detection information corresponding to the bioelectric signal. The circuit shown in FIG. 4 may be used for the detection circuits shown in FIG. 2 and FIG. 3, or may be used for the detection and heating circuit 110 shown in FIG. 1, or may be used for a detection and heating circuit shown in FIG. 9, FIG. 11, or FIG. 16, and is configured to obtain the detection information corresponding to the bioelectric signal.

As shown in FIG. 4, the detection circuit 400 includes a direct current or working frequency suppression module 410, a signal amplification module 420, an analog filtering module 430, a digital-to-analog conversion module 440, and a digital filtering module 450.

The direct current or working frequency suppression module 410 is configured to block direct current and working frequency interference from other circuits. The signal amplification module 420 is configured to amplify the received bioelectric signal, and send the amplified bioelectric signal to the analog filtering module 430. The analog filtering module 430 is configured to receive the bioelectric signal amplified by the signal amplification module 420, perform filtering processing on the amplified bioelectric signal, and send the filtered bioelectric signal to the analog-to-digital conversion module 440. The analog-to-digital conversion module 440 is configured to receive the bioelectric signal filtered by the analog filtering module 430, convert the filtered bioelectric signal from an analog signal to a digital signal, and send the digital bioelectric signal to the digital filtering module 450. The digital filtering module 450 is configured to receive the digital bioelectric signal sent by the analog-to-digital conversion module 440, and perform filtering processing on the digital bioelectric signal in digital domain to obtain a digitally filtered bioelectric signal. The digitally filtered bioelectric signal may be used as the detection information corresponding to the bioelectric signal of the user, and sent to subsequent modules, so that switching of a working mode of the detection and heating material and parameters in the working mode can be subsequently controlled based on the detection information.

The direct current or working frequency suppression module 410 can block direct current and working frequency interference from other circuits, for example, block 50 Hz working frequency interference, or for another example, block a direct current signal that is applied to the detection and heating material and used for heating, so that no interference is caused to bioelectric signal detection. In this case, the direct current or working frequency suppression module 410 implements a circuit protection function, and may be considered as a circuit protection apparatus.

It should be noted that, alternatively, the modules shown in FIG. 4 may be partially omitted, that is, a function of the detection circuit 400 is to perform certain preprocessing on the bioelectric signal, but the processing procedure may be different. For example, the direct current or working frequency suppression module 410 may be omitted. For another example, analog-to-digital conversion may not be performed, and subsequent operations may be performed directly based on the analog bioelectric signal. Details are not described herein again. FIG. 4 is merely an example of a detection circuit according to this embodiment of this application. Without creative efforts, a person skilled in the art may further obtain another circuit structure having a similar function.

It can be learned that, in this embodiment of this application, under control of the detection and heating circuit, the detection and heating material is enabled to work in two working modes, that is, the detection and heating material has both the bioelectric signal detection function and the heating function. To implement switching between the two working modes, the control circuit 120 may be set to control the detection and heating circuit 110. The following describes the control circuit 120.

The control circuit 120 is configured to instruct the detection and heating circuit 110 to control the detection and heating material to work in the detection mode or the heating mode.

Optionally, the control circuit 120 instructs the detection and heating circuit 110 to set the working mode of the detection and heating material to the detection mode. In this working mode, the detection and heating circuit 110 obtains a first bioelectric signal of the user by using the detection and heating material, and may perform certain processing on the first bioelectric signal to obtain corresponding first detection information, and then send the first detection information to the control circuit 120. The control circuit 120 obtains the first detection information, generates second detection information based on the first detection information, and sends the second detection information to the target device by using the transceiver circuit 130.

Optionally, the first detection information may be a bioelectric signal obtained after processing is performed, for example, may be a bioelectric signal obtained after processing is performed by using the detection circuit shown in FIG. 4.

Optionally, the second detection information may be the first detection information itself, or certain processing such as decomposition or feature extraction may be performed on the first detection information to obtain the second detection information.

Optionally, a signal decomposition module may be set to decompose the first detection information. For example, a signal decomposition module may be set after the digital filtering module 450 of the detection circuit shown in FIG. 4, and is configured to obtain the first detection information (for example, a bioelectric signal processed by the detection circuit shown in FIG. 4) and decompose the first detection information to obtain a decomposed signal.

Optionally, the first detection information may be decomposed by using a blind signal separation method, to obtain a decomposed signal of the first detection information.

For example, an ICA method is used to decompose the first detection information to obtain source signal components of the first detection information, where the components may correspond to an electroencephalogram signal, an electro-oculogram signal, an electromyography signal, and the like. In this way, the electroencephalogram signal, the electro-oculogram signal, and the electromyography signal are obtained through decomposition.

Optionally, the decomposed signal of the first detection information (for example, the electroencephalogram signal, the electro-oculogram signal, or the electromyography signal obtained through decomposition) may be used as the second detection information.

Optionally, the control circuit 120 may further perform feature extraction on the decomposed signal of the first detection information, to obtain a feature signal of the decomposed signal of the first detection information.

Optionally, when the control circuit 120 performs feature extraction on the decomposed signal of the first detection information, a method of energy sum ratio analysis in a frequency domain analysis method may be used to extract the feature signal of the decomposed signal such as the electroencephalogram signal, the electro-oculogram signal, or the electromyography signal obtained by decomposing the first detection information, and the feature signal may be used as the second detection information.

In summary, it can be learned that the second detection information may be a bioelectric signal (for example, the first detection information) obtained after the bioelectric signal obtained by the detection and heating material is processed, or may be a decomposed signal obtained after the processed bioelectric signal (for example, the first detection information) is decomposed, or may be a feature signal obtained after feature extraction is performed on the decomposed signal.

First, signal segmentation is performed on the electroencephalogram signal, the electro-oculogram signal, and the electromyography signal.

For example, signal segmentation is performed on the electroencephalogram signal, the electro-oculogram signal, and the electromyography signal separately, and a data length of each signal segment is 7500 points. For example, a sampling frequency may be set to 250 Hz, capture duration may be set to 30 seconds (s), signal data of 25 minutes at each stage is selected for feature extraction, and a ratio of energy of each feature at each fatigue stage to an energy sum is calculated.

Then the electroencephalogram signal is decomposed by using a wavelet decomposition method, to obtain energy ratios of $\alpha$, $\beta$, $\theta$, and $\delta$ waves. For example, wavelet decomposition is performed on the electroencephalogram signal by using a db4 wavelet base, to calculate ratios of energy of the $\alpha$ wave (8 Hz to 13 Hz), $\beta$ wave (13 Hz to 30 Hz), $\theta$ wave (4 Hz to 7 Hz), and $\delta$ wave (1 Hz to 4 Hz) to an energy sum within a frequency range of 1 Hz to 30 Hz separately.

Similarly, wavelet decomposition is performed on the electromyography signal and the electro-oculogram signal by using the wavelet base, and ratios of energy thereof to the energy sum within the frequency range of 1 Hz to 30 Hz are calculated.

A specific implementation may be: inputting a segmented signal Wm of the electroencephalogram signal into a matlab wavelet decomposition tool library function, where W represents the segmented signal of the electroencephalogram signal, and m represents a signal number input to the tool library function and a value range of m is {1, 2, ..., p}, where p is a positive integer and represents a total quantity of signals input to the tool library function. The tool library function may be, for example, wavedec_2, and an input of the function wavedec_2 includes the input signal Wm, a decomposition level, and a wavelet base type. It should be noted that the decomposition level is related to a frequency band type of a brain wave to be decomposed. Therefore, 4 may be selected for the decomposition level, and db4 may be selected for the wavelet base type. In this case, an output of wavedec_2 is decomposition coefficients at all levels, of $\alpha$, $\beta$, $\theta$, and $\delta$ wave signals in the corresponding electroencephalogram signal, and lengths of the decomposition coefficients at all the levels, that is, sizes.

Wavelet decomposition is localized time (space) frequency analysis. Wavelet decomposition is to perform multi-scale refinement on a signal step by step through scaling and translation operations, and finally achieve time subdivision at a high frequency and frequency subdivision at a low frequency. It can automatically adapt to requirements of time-frequency signal analysis, thereby focusing on any detail of the signal, and resolving a problem of difficulty in Fourier transform.

How to detect a signal accurately in noise is always a concern in the field of signal processing. Wavelet decomposition is a time-scale signal analysis method. Because wavelet decomposition has a multi-resolution analysis characteristic and a good time-frequency localization characteristic, it can decompose various time-varying signals effectively, thereby separating signals from noise properly, and achieving a satisfactory denoising effect.

Therefore, an energy ratio can be obtained through calculation. Referring to formulas (1) and (2), Di (k) is a $k^{th}$ wavelet coefficient in a level-i frequency band after decomposition; n is a quantity of data of a level-i wavelet coefficient; Es is an energy sum of bioelectric signals corresponding to a frequency band at each level; and N is a total quantity of levels, that is, a total quantity of frequency bands obtained through division.

A formula for calculating the energy ratio is as follows:

$$\mu_i = \frac{\sum_{k=1}^{n} \left| D_i\left(k\right) \right|^2}{E_S} , \tag{1}$$

$$E_s = \sum_{i=1}^{N} \left| D_i\left(k\right) \right|^2 , \tag{2}$$

where $\mu_i$ is a ratio of energy of the level-i frequency band after decomposition to the energy sum, and different frequency bands correspond to different types of bioelectric signals; $D_i$ (k) is the $k^{th}$ wavelet coefficient in the level-i frequency band after decomposition; n is the quantity of data of the level-i wavelet coefficient; Es is the energy sum of bioelectric signals corresponding to the frequency band at each level; and N is the total quantity of levels, that is, the total quantity of frequency bands obtained through division.

The transceiver circuit 130 may be configured to send the second detection information to the target device 200 (for example, a cloud device or a terminal), where the second detection information corresponds to the first detection information; and the transceiver circuit 130 may be further configured to receive first instruction information corresponding to the second detection information, and send the first instruction information to the control circuit 120.

It should be understood that the first detection information may be a processed bioelectric signal obtained by using the detection circuit shown in FIG. 4. The second detection information may be the same as the first detection information, or may be the decomposed signal of the first detection information, or may be the feature signal of the decomposed signal of the first detection information. All the foregoing may be obtained by using the foregoing method, and details are not described herein again.

It should also be understood that, what is available between the transceiver circuit 130 and the target device 200 may be a wired transmission communication mode, or may be an electrical connection using a wire, or may be a wireless transmission communication mode. Alternatively, the transceiver circuit 130 may be a transceiver, an interface circuit, a communications unit, a communications module, a transceiver unit, a transceiver module, or the like. Details are not described herein.

Optionally, the two working modes of the detection and heating material may be controlled according to a preset rule. It should be noted that the process may be completed by the target device 200 (for example, a cloud device or a terminal) in cooperation with the intelligent control apparatus 100, or a corresponding module may be set in the control circuit 120 and the process is completed by the intelligent control apparatus 100. In the following description, it is assumed that the target device 200 cooperates with the intelligent control apparatus 100 to complete the process.

Optionally, the target device 200 may be, for example, a device that has functions such as computing and analysis, such as a cloud device or a terminal.

Optionally, the target device 200 may include the transceiver 210 and the processor 220. The transceiver 210 is configured to cooperate with the transceiver circuit 130 to implement information interaction between the intelligent control apparatus 100 and the target device 200; and the processor 220 is configured to generate corresponding indication information based on detection information that is obtained from the intelligent control apparatus 100 by using the transceiver 210, and send the indication information to the intelligent control apparatus 100 by using the transceiver 210.

It should be understood that the transceiver 210 may also be a transceiver circuit, an interface circuit, a communications unit, a communications module, a transceiver unit, a transceiver module, or the like, and may connect to or communicate with the intelligent control apparatus 100 in a wired or wireless manner. Details are not described herein again.

Optionally, the two working modes may be controlled by setting parameters of the detection mode and the heating mode, such as working duration and the heating temperature. For example, the working duration of the detection mode may be set to 30 seconds (s), the heating duration of the heating mode is set to 15 minutes (min), the heating temperature is 40°C, and heating is not performed when bioelectric signal detection is performed, or bioelectric signal detection is not performed when heating is performed. In other words, the detection and heating circuit 110 may be instructed to control the working mode of the detection and heating material, and the detection and heating circuit 110 is instructed to control the parameters (for example, the working duration and the heating temperature) of the detection and heating material in the working mode, thereby controlling the two working modes. In this example, the foregoing setting may be implemented by the control circuit 120. For example, the control circuit 120 may instruct the detection and heating circuit 110 to turn on the switch between the detection and heating material and the detection circuit and to turn off the switch between the detection and heating material and the heating circuit, so that the detection and heating material works in the detection mode. When preset working duration (for example, 30s) of the detection mode is reached, the control circuit 120 instructs the detection and heating circuit to turn off the switch between the detection and heating material and the detection circuit and to turn on the switch between the detection and heating material and the heating circuit, so that the detection and heating material works in the heating mode. When preset working duration (for example, 15 minutes) of the heating mode is reached, the control circuit 120 instructs the detection and heating circuit to turn off the switch between the detection and heating material and the heating circuit, so that the detection and heating material terminates the heating mode. In the heating mode, the control circuit 120 may further instruct the detection and heating circuit 110 to set an electrical signal parameter value applied to the detection and heating material (for example, set an applied voltage 5 V). Therefore, by controlling strength of the electrical signal applied to the detection and heating material, the heating temperature of the detection and heating material working in the heating mode is controlled.

Optionally, a temperature controller, a temperature control circuit, a temperature control module, or the like (hereinafter collectively referred to as a temperature controller) may also be set, so that the heating temperature of the detection and heating material can be stabilized within a temperature range in the heating mode. In other words, heating may be performed within a floating range of a specified heating temperature. For example, when the heating temperature is set to 40°C, the temperature controller may be used to stabilize the heating temperature within a range of $40\pm0.5$°C. It should be understood that a structure of the temperature controller is not limited, but may be selected as required by a person skilled in the art. The setting of the floating range is merely an example, and is not limited either. Details are not described herein. It should also be understood that the temperature controller may be disposed in the control circuit 120, or may be disposed in the detection and heating circuit 110, or may be independent of the control circuit and the detection and heating circuit.

The temperature controller, also referred to as an automatic temperature regulator, is usually implemented by using a circuit. A core of this circuit is to set a temperature threshold by using a heat-sensitive element, to trigger turn-on or turn-off of a switch. Temperature adjustments may also be divided into fine-granularity level adjustments. For example, there are three levels: Temperature T1 > Temperature T2 > Temperature T3. If a currently specified temperature level is the temperature T3, and a temperature sensor in the temperature controller detects that a current temperature Tc is lower than the temperature T3, that is, Tc < T3, a temperature controller control switch is connected to a heating module (for example, the heating circuit shown in FIG. 2 or FIG. 3). When the temperature Tc detected by the temperature sensor is higher than or equal to the temperature T2, that is, Tc $\geq$ T2, the temperature controller switch is connected to a detection module (for example, the detection circuit shown in FIG. 2 or FIG. 3 or the detection circuit shown in FIG. 4), and a bioelectric signal detected by the detection module is transmitted to a processing apparatus (for example, the target device shown in FIG. 1 or the control circuit shown in FIG. 11), so that the processing apparatus can obtain a new policy for adjusting the state of the user, for example, obtain a new temperature level and apply the new level to the temperature controller.

Optionally, the temperature controller may be electronic or mechanical.

Optionally, the temperature controller may be programmed to use a program to control and send various signals to the heating circuit of the conducting and heating material, to achieve an objective of controlling the connection/disconnection of the heating circuit and implement a function of a switch.

Division of circuits, modules, units, and the like in this embodiment of this application is merely logical division, and another logical division manner may also be used. The circuit in this embodiment of this application may alternatively be implemented in a form other than the circuit. For example, the transceiver circuit may alternatively be a communications unit. However, for ease of description, the circuit is mainly used as an example for description, and details are not described.

Optionally, switching the working mode of the detection and heating material to the heating mode, and the heating duration and/or the heating temperature in the heating mode may be further controlled based on the bioelectric signal detected in the detection mode. For example, the working duration of the detection mode is set to 30s, and after the detection ends, the detected bioelectric signal is compared with a preset value range of the bioelectric signal, and the heating duration and/or the heating temperature of the detection and heating material in the heating mode are/is set based on a comparison result.

In an implementation, the control circuit 120 may instruct the detection and heating circuit 110 to set the working mode of the detection and heating material to the detection mode; in the detection mode, the detection and heating circuit 110 obtains the first bioelectric signal of the user by using the detection and heating material, and processes the first bioelectric signal to obtain the first detection information; the transceiver circuit 130 sends, to the transceiver 210, the second detection information corresponding to the first detection information; the transceiver 210 receives the second detection information, and sends the second detection information to the processor 220; the processor 220 compares the second detection information with a preset value range of the second detection information, and generates first instruction information based on a comparison result, where the first instruction information is used to indicate the heating duration and/or the heating temperature of the detection and heating material in the heating mode; the transceiver 210 sends the first instruction information to the transceiver circuit 130; the transceiver circuit 130 receives the first instruction information, and sends the first instruction information to the control circuit 120; the control circuit 120 instructs, according to the first instruction information, the detection and heating circuit 110 to switch the working mode of the detection and heating material to the heating mode; and the control circuit 120 further instructs, according to the first instruction information, the detection and heating circuit 110 to control the heating duration of the detection and heating material by controlling duration of the electrical signal applied to the detection and heating material, and control the strength of the electrical signal applied to the detection and heating material, so that the heating temperature of the detection and heating material is the temperature indicated by the first instruction information.

In another implementation, the control circuit 120 may further control switching of the two working modes autonomously. For example, it may be specified that when a detected bioelectric signal is not within a preset range, the working mode is switched to the heating mode, and fixed heating duration is set, for example, set to 5 minutes (min); after 5 minutes, the working mode is switched to the detection mode, and detection is performed again; if a detected bioelectric signal is within the preset range, the process is stopped; if a detected bioelectric signal is not within the preset range, the process of heating for 5 minutes is repeated, and the process is not stopped until a detected bioelectric signal is within the preset range. It should be understood that this implementation may be considered as a process of alternate detection and heating, where the process is not stopped until a value is within the preset range. However, there is also another implementation. For example, in a process of repeating heating for fixed duration, the heating temperature may be adjusted based on a difference between a detected bioelectric signal and the preset range.

For further example, it may be specified that when the detected bioelectric signal is within the preset value range, heating intervention is not performed; or both the heating temperature and duration are set to be relatively small, and when the detected bioelectric signal is not within the preset value range, heating intervention is performed. In addition, the heating duration and/or temperature may be adjusted based on the difference between the detected bioelectric signal and the preset range. This example may also be completed by the target device 200 in cooperation with the intelligent control apparatus 100, or may be completed independently by the intelligent control apparatus 100. Because the process is similar, details are not described again.

For another example, the working duration of the detection mode is set, and after the detection ends, it is determined, based on the detected first bioelectric signal, that the user is in a relatively good state, for example, only slight fatigue or only slight anxiety. In this case, the duration of the heating mode may be set to a relatively short value, and the heating temperature is also set to a relatively low temperature. Further, when the detection is performed again, it is determined, based on a first bioelectric signal detected again, that the user is in a relatively poor state, that is, great fatigue, high tension, or the like. In this case, the heating duration of the heating mode may be set to a relatively large value, and the heating temperature is also set to a relatively high temperature, or only the heating duration of the heating mode is prolonged without changing the heating temperature, or only the heating temperature is increased without changing the heating duration. It should be understood that in the foregoing process of this example, only one of the heating duration and the heating temperature may be set, or both may be set. The heating temperature may be adjusted by adjusting the strength of the electrical signal such as the current and/or the voltage applied to the detection and heating material. In an implementation of this example, the adjustment may be completed by the target device 200 in cooperation with the intelligent control apparatus 100.

Optionally, the control circuit 120 may instruct the detection and heating circuit 110 to set the working mode of the detection and heating material to the detection mode; in the detection mode, the detection and heating circuit 110 obtains the first bioelectric signal of the user by using the detection and heating material, and processes the first bioelectric signal to obtain the first detection information; the transceiver circuit 130 sends, to the transceiver 210, the second detection information corresponding to the first detection information; the transceiver 210 receives the second detection information, and sends the second detection information to the processor 220; the processor 220 determines a state category of the user based on the second detection information, determines a corresponding improvement solution based on the state category, and generates first instruction information corresponding to the improvement solution, where the improvement solution includes the heating duration and/or the heating temperature of the detection and heating material working in the heating mode; the transceiver 210 sends the first instruction information to the transceiver circuit 130; the transceiver circuit 130 receives the first instruction information, and sends the first instruction information to the control circuit 120; the control circuit 120 instructs, according to the first instruction information, the detection and heating circuit 110 to switch the working mode of the detection and heating material to the heating mode; and the control circuit 120 further instructs, according to the first instruction information, the detection and heating circuit 110 to perform the improvement solution.

In the process of determining the state category of the user based on the second detection information, the processor 220 may perform different processing based on different cases of the second detection information.

When the second detection information is the first detection information, the processor 220 decomposes the second detection information to obtain a decomposed signal, then performs feature extraction on the decomposed signal to obtain a feature signal, and then determines the state category of the user based on the feature signal. When the second detection information is the decomposed signal of the first detection information, the processor 220 performs feature extraction on the second detection information to obtain a feature signal, and then determines the state category of the user based on the feature signal. When the second detection information is a feature signal of the first detection information, the processor 220 directly determines the state category of the user based on the second detection information.

Optionally, when determining the state category of the user based on the extracted feature signal, the processor 220 may analyze and determine the state category by using the following method.

It should be noted that the state category of the user is used to indicate the state of the user, and the state of the user may be various mental states or physiological states such as a fatigue state, an anxiety state, a sleep state, a depression state, a stress state, or a tension state. For example, the state category may be classified into a normal state, a slightly abnormal state, a moderately abnormal state, and a severely abnormal state. When corresponding to a state, for example, corresponding to the fatigue state, the fatigue state category may be classified into non-fatigue, general fatigue, and great fatigue.

Optionally, the processor 220 may compare the bioelectric signal (for example, the electroencephalogram signal, the electro-oculogram signal, or the electromyography signal) of the user with a normal value or a preset value, classify an abnormal level based on a difference, and further determine the state category of the user. For example, both the electroencephalogram signal and the electromyography signal determined based on the detected bioelectric signal differ greatly from the normal value; therefore, it is inferred that the user is in the severely abnormal state, such as great fatigue. For ease of understanding, the following uses the fatigue state of the user as an example for description. Therefore, it may be understood that in the following method, the fatigue state may also be replaced with another state, for example, the user state such as the anxiety state or the depression state.

Optionally, the fatigue category of the user may be classified, for example, classified into non-fatigue, general fatigue, and great fatigue. Further, a fatigue degree of the user may be classified, for example, classified into slight fatigue, moderate fatigue, or severe fatigue. Alternatively, a fatigue degree of the user may be classified into levels. For example, the fatigue degree is classified into the following levels: 1-non-fatigue, 2-general fatigue, and 3-great fatigue.

It should be understood that the fatigue category, the fatigue degree, and the fatigue degree level are all descriptions of the fatigue state of the user, and belong to different descriptions of a same thing. In this embodiment of this application, the fatigue category is used as an example for description.

Optionally, value ranges of corresponding bioelectric signals of different fatigue categories may be set; the detected first bioelectric signal is processed to obtain the first detection information; the first detection information is compared with a specified value range; and the fatigue category of the user is determined based on a comparison result. Alternatively, the second detection information corresponding to the first detection information may be obtained; the second detection information is compared with a preset range corresponding to the second detection information; and the fatigue category of the user is determined based on a comparison result. Because the first bioelectric signal corresponds to the first detection information and the first detection information corresponds to the second detection information, it may be considered that a determined correspondence exists between the first bioelectric signal and the second detection information.

Optionally, a fatigue category classification model may be further established. After the first bioelectric signal is detected, the second detection information corresponding to the first bioelectric signal is obtained based on the first bioelectric signal, and the fatigue category of the user is determined based on the second detection information and the fatigue category classification model.

Optionally, the foregoing fatigue category classification model may be obtained through training by using a machine learning method. For example, a support vector machine (support vector machine, SVM) and a convolutional neural network (convolutional neural network, CNN) are used as examples for description.

Optionally, the foregoing SVM model may be trained by using the following method, and parameters of the SVM model may be determined and updated, to improve accuracy when the model is used to recognize a fatigue category.

A third bioelectric signal of the user is obtained, where the third bioelectric signal may be, for example, a bioelectric signal captured within 30s; the foregoing method is used to perform processing, decomposition, and feature extraction on the third bioelectric signal to obtain an electroencephalogram signal, an electro-oculogram signal, and an electromyography signal of the third bioelectric signal; and a six-dimensional feature matrix including ratios of energy of $\alpha$, $\beta$, $\theta$, and $\delta$ waves of the electroencephalogram signal, the electro-oculogram signal, and the electromyography signal to an energy sum within the frequency range is imported into the SVM, and SVM model parameters with higher recognition accuracy are obtained through training.

Optionally, a classification probability result of the fatigue category is obtained by using data of a 30s segmented signal, and the SVM model parameters with high recognition accuracy are obtained through training.

Optionally, the SVM uses a radial basis kernel function as an activation function, and selects, based on experience, to perform recognition when $\gamma$ is 8.0 and a penalty factor C is 10.0, to achieve higher recognition accuracy.

It should be understood that, the feature extraction may alternatively not be performed, and the electroencephalogram signal, the electromyography signal, and the electro-oculogram signal that are obtained through decomposition are directly used to perform SVM model parameter training.

When the fatigue category classification model obtained through training is used to recognize the fatigue category, the first bioelectric signal of the user is detected to obtain the second detection information corresponding to the first bioelectric signal; the feature signal such as the ratio to the energy sum, determined based on the second detection information, is imported into the SVM model obtained through training; a probability of a fatigue category corresponding to the first bioelectric signal is determined; and a classification probability of the fatigue category corresponding to the bioelectric signal in a time period is output.

It should be noted that both the first bioelectric signal and the third bioelectric signal may be obtained by using the detection and heating circuit 110 provided in this embodiment of this application. The first bioelectric signal refers to a bioelectric signal captured for determining the fatigue state of the user, and the third bioelectric signal refers to a bioelectric signal captured for establishing the fatigue category classification model. The two bioelectric signals are distinguished only based on purposes.

It should also be understood that when the state of the user is another state such as the sleep state, the corresponding SVM model may also be obtained through training by using the foregoing method, and a trained state classification model is used to determine other state categories such as the sleep state category of the user based on the second detection information that is determined based on the captured first bioelectric signal. Details are not described herein.

Optionally, a deep learning method may be further used to obtain the user fatigue category classification model through training. For example, the convolutional neural network may be used to perform training. The following describes a training process of the user fatigue category classification model by using the convolutional neural network as an example.

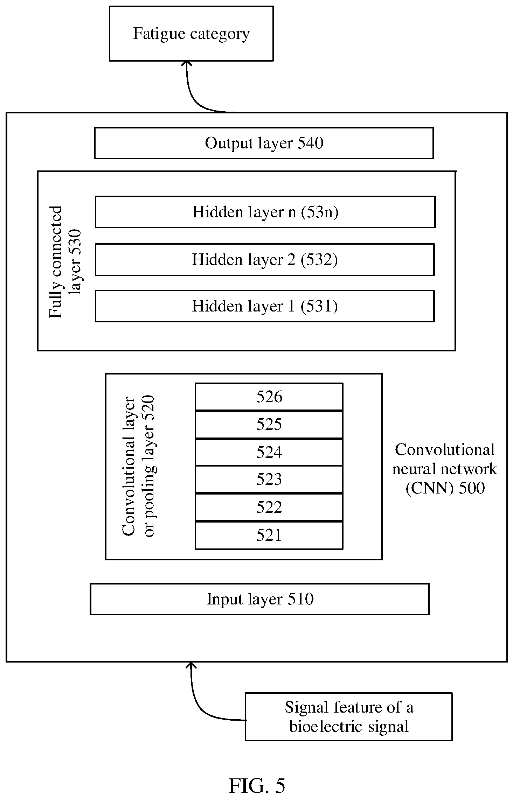

In this embodiment of this application, a convolutional neural network with a structure shown in FIG. 5 may be used to train the user fatigue category classification model. FIG. 5 is a schematic diagram of a structure of a convolutional neural network according to an embodiment of this application. As shown in FIG. 5, a convolutional neural network 500 may include an input layer 510, a convolutional layer or pooling layer 520 (the pooling layer is optional), and a fully connected layer 530. The input layer 510 may obtain a feature signal of the third bioelectric signal, and submit the feature signal of the third bioelectric signal to the convolutional layer or pooling layer 520 and the subsequent fully connected layer 530 for processing, so that the fatigue category of the user can be obtained.

As shown in FIG. 5, for example, the convolutional layer or pooling layer 520 may include layers 521 to 526. For example, in an example, the layer 521 is a convolutional layer, the layer 522 is a pooling layer, the layer 523 is a convolutional layer, the layer 524 is a pooling layer, the layer 523 is a convolutional layer, and the layer 526 is a pooling layer. In another example, the layers 521 and 522 are convolutional layers, the layer 523 is a pooling layer, the layers 524 and 523 are convolutional layers, and the layer 526 is a pooling layer. In other words, output of a convolutional layer may be used as input for a subsequent pooling layer, or may be used as input for another convolutional layer, to continue to perform a convolution operation.

The fully connected layer 530 may include a plurality of hidden layers (531, and 532 to 53n shown in FIG. 5) and an output layer 540. Parameters included in the plurality of hidden layers may be obtained by performing pre-training based on related training data of a specific task type. For example, the task type may include signal recognition or signal classification.

The output layer 540 is located after the plurality of hidden layers in the fully connected layer 530, that is, it is a last layer of the entire convolutional neural network 500. The output layer 540 has a loss function similar to a classification cross entropy, and the loss function is specifically used to calculate a prediction error. Once forward propagation of the entire convolutional neural network 500 (as shown in FIG. 5, propagation from 510 to 540 is forward propagation) is completed, back propagation (as shown in FIG. 5, propagation from 540 to 510 is back propagation) starts to update weight values and deviations of the layers mentioned above, to reduce a loss of the convolutional neural network 500 and an error between a result output by the convolutional neural network 500 by using the output layer and an ideal result.

Optionally, the following method may be used to train the user fatigue category classification model by using the convolutional neural network shown in FIG. 5. To be specific, after feature signals of the electroencephalogram signal, the electro-oculogram signal, and the electromyography signal of the third bioelectric signal are processed by the input layer 510, the convolutional layer or pooling layer 520, and the fully connected layer 530, corresponding fatigue categories may be obtained. This is equivalent to completing classification of user fatigue.

First, the third bioelectric signal of the user is obtained, and processing such as denoising is performed on the third bioelectric signal to obtain detection information corresponding to the third bioelectric signal, and the detection information is decomposed based on a subspace decomposition algorithm ICA to obtain a decomposed signal of the detection information (for example, the electroencephalogram signal, the electro-oculogram signal, and the electromyography signal). A multi-dimensional signal formed by the decomposed signal or the feature signal of the decomposed signal is input into the convolutional neural network together with a label (which may be a fatigue category to which a doctor has added a classification label) corresponding to the multi-dimensional signal. Specifically, each type of signal of the decomposed signal is segmented, and a data length of each segment may be 7500 points (sampling at a 250 Hz frequency and capturing for 30s). For example, when the electroencephalogram signal, the electro-oculogram signal, and the electromyography signal all exist, a three-dimensional signal feature matrix of the electroencephalogram signal, the electro-oculogram signal, and the electromyography signal is formed, and the signal feature matrix is input into the CNN together with a label corresponding to the signal feature matrix. An iterative optimization solution is performed based on a stochastic gradient descent algorithm. Finally, convolution kernel parameters corresponding to convergence of an objective function and parameters in the fully connected layer are obtained. Finally, a softmax layer may be used as a recognition layer, and a quantity of corresponding output ports is determined based on a quantity of fatigue categories that need to be distinguished.

At the foregoing training stage, various parameters in the convolutional layer, the pooling layer, the fully connected layer, and the classification model need to be determined, to ensure high accuracy of subsequent target recognition. The classification probability result of the fatigue category may be obtained by using data of the 30s segmented signal.

It should be understood that the 7500 points (sampling at the 250 Hz frequency and capturing for 30s) selected for the foregoing data length are merely an example. Alternatively, another sampling frequency and sampling duration may be selected. In addition, in the training process, the ratio to the energy sum is only one feature of the signal, and other features, such as power spectrum estimation of various types of signals, may also be selected for model training, to obtain the fatigue category classification model.

When the fatigue category classification model obtained through the training is used to recognize the fatigue category, the first bioelectric signal of the user is detected, a multi-dimensional signal formed by the electroencephalogram signal, the electro-oculogram signal, and the electromyography signal that are obtained by preprocessing the first bioelectric signal is input into the convolutional neural network for which the model parameters of each module are determined at the training stage, and a fatigue category recognition probability output by a softmax classification model of the convolutional neural network by performing calculation is obtained.

The foregoing process of training the fatigue category classification model and recognizing the fatigue category of the user may be performed by the processor 220. However, in another implementation, alternatively, the foregoing process may be performed in the intelligent control apparatus 100.

In the foregoing process, by using the training method of the neural network, the fatigue category of the user is determined based on the detected bioelectric signal and a training model, so that the fatigue state of the user is determined more accurately.

Optionally, after determining the fatigue category of the user according to the foregoing method, the processor 220 may further determine a corresponding improvement solution based on the foregoing fatigue category, for example, may determine the working mode of the detection and heating material and the heating duration and/or the heating temperature in the heating mode.

For example, when the determined fatigue category is great fatigue, the heating duration in the improvement solution may be prolonged, and the heating temperature in the improvement solution may be increased.

It should be noted that, in actual application, the intelligent control apparatus provided in this embodiment of this application may further set an initial mode or a power-on mode. For example, the power-on mode may be set to the detection mode. When the user is using the intelligent control apparatus, detection is performed on the user after power-on, and then the heating temperature and/or the heating duration are/is determined based on the detected bioelectric signal. For another example, the initial mode or the power-on mode may alternatively be set to a mode that can be manually selected and switched by the user. For example, a mode selection module may be set, so that the user can autonomously select the initial mode or the power-on mode.

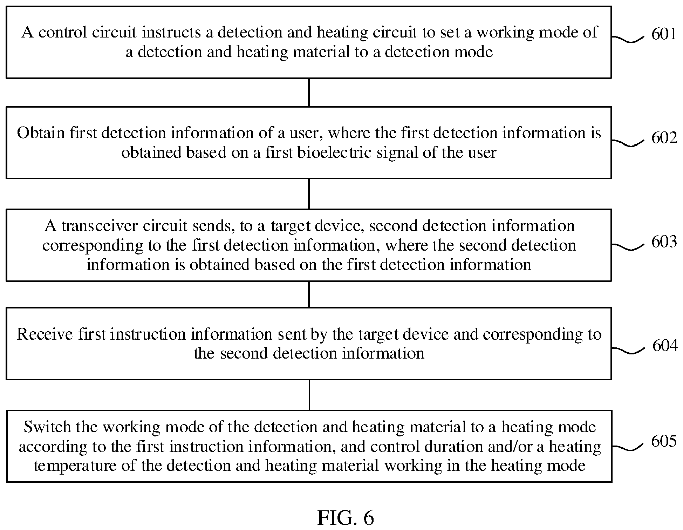

With reference to FIG. 6, the following illustrates a process of intelligently improving a state of a user by using the intelligent control apparatus shown in FIG. 1. FIG. 6 is a schematic flowchart of a control method for an intelligent control apparatus according to an embodiment of this application. The following describes steps shown in FIG. 6.

601. A control circuit instructs a detection and heating circuit to set a working mode of a detection and heating material to a detection mode.

Optionally, the control circuit 120 instructs the detection and heating circuit 110 to set the working mode of the detection and heating material to the detection mode.

602. Obtain first detection information of a user, where the first detection information is obtained based on a first bioelectric signal of the user.

Optionally, in the detection mode, the detection and heating circuit 110 obtains the first bioelectric signal of the user by using the detection and heating material. The detection and heating circuit 110 processes the first bioelectric signal to obtain the first detection information corresponding to the first bioelectric signal.

603. A transceiver circuit sends, to a target device, second detection information corresponding to the first detection information, where the second detection information may be the first detection information itself, or may be a decomposed signal of the first detection information, or may be a feature signal of a decomposed signal of the first detection information.

Optionally, the transceiver circuit 130 sends, to the target device 200, the second detection information corresponding to the first detection information, where the second detection information may be the first detection information itself, or may be the decomposed signal of the first detection information, or may be the feature signal of the decomposed signal of the first detection information.

604. Receive first instruction information sent by the target device and corresponding to the second detection information.

Optionally, the transceiver circuit 130 receives the first instruction information, and sends the first instruction information to the control circuit 120.

Optionally, the target device may obtain the first instruction information by using the following steps.

Step 1: The transceiver 210 receives the second detection information, and sends the second detection information to the processor 220.

Step 2: The processor 220 analyzes the second detection information, to determine the first instruction information corresponding to the second detection information.

Optionally, the processor 220 may determine the state category of the user based on the second detection information by using the related method provided above, and then determine the first instruction information based on the state category.

Optionally, the processor 220 may further determine an improvement solution based on the state category, and then generate indication information used to indicate the solution. For example, the processor 220 determines, by analyzing the second detection information, that a fatigue category of the user in the time period is "category 1", and selects, based on a mapping relationship in Table 2, a mitigation solution in which "a heating temperature is 38°C, massage strength is weak, and operation duration of both heating and massage is 10 minutes"; and accordingly, the target device generates the first instruction information used to indicate the foregoing mitigation solution.

Step 3: The transceiver 210 sends the first instruction information to the transceiver circuit 130.

605. Switch the working mode of the detection and heating material to a heating mode according to the first instruction information, and control duration and/or a heating temperature of the detection and heating material working in the heating mode.

Optionally, the control circuit 120 instructs, according to the first instruction information, the detection and heating circuit 110 to set the working mode of the detection and heating material to the heating mode.

Optionally, the control circuit 120 instructs, according to the first instruction information, the detection and heating circuit 110 to control the heating duration and/or the heating temperature of the detection and heating material in the heating mode.

It should be noted that the foregoing steps may be performed by using the foregoing related method, and the foregoing steps may not be completely performed. For example, the control circuit 120 may only set two working modes of the detection and heating material to perform detection and heating alternately based on fixed duration, and instruct the detection and heating material 110 to perform corresponding actions. In this case, a process such as determining the state category of the user is not required. This example has been described above, and details are not described herein again.

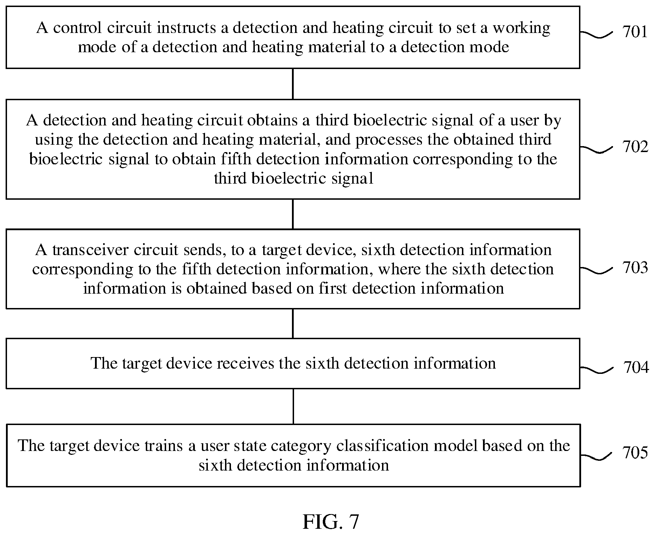

With reference to FIG. 7, the following illustrates a process of obtaining a user state category classification model by using the intelligent control apparatus and the target device shown in FIG. 1. FIG. 7 is a schematic flowchart of a method for obtaining a user state category classification model according to an embodiment of this application. The following describes steps in FIG. 7.

701. A control circuit instructs a detection and heating circuit to set a working mode of a detection and heating material to a detection mode.

Optionally, the control circuit 120 instructs the detection and heating circuit 110 to set the working mode of the detection and heating material to the detection mode.

702. The detection and heating circuit obtains a third bioelectric signal of a user by using the detection and heating material, and processes the obtained third bioelectric signal to obtain fifth detection information corresponding to the third bioelectric signal.

Optionally, in the detection mode, the detection and heating circuit 110 obtains the third bioelectric signal of the user by using the detection and heating material, and processes the obtained third bioelectric signal to obtain the fifth detection information corresponding to the third bioelectric signal (for example, the third bioelectric signal processed by using the foregoing detection circuit 400).

703. A transceiver circuit sends, to a target device, sixth detection information corresponding to the fifth detection information, where the sixth detection information is obtained based on first detection information.

Optionally, the transceiver circuit 130 sends, to the target device 200, the sixth detection information corresponding to the fifth detection information, where the sixth detection information may be the fifth detection information itself, or may be a decomposed signal of the fifth detection information, or may be a feature signal of a decomposed signal of the fifth detection information.

704. The target device receives the sixth detection information.

Optionally, the transceiver 210 receives the sixth detection information, and sends the sixth detection information to the processor 220.

705. The target device trains a user state category classification model based on the sixth detection information.

Optionally, the processor 220 trains the user state category classification model based on the sixth detection information.

Optionally, the machine learning method described above may be used to train the user state category classification model by using the sixth detection information (that is, the processed third bioelectric signal, the decomposed signal of the third bioelectric signal, or the feature signal of the decomposed signal of the third bioelectric signal).

It should be noted that the foregoing method may also be used to update the user category classification model.

With reference to FIG. 8, the following illustrates a process of updating a user state category classification model by using the intelligent control apparatus and the target device shown in FIG. 1. FIG. 8 is a schematic flowchart of a method for updating a user state category classification model according to an embodiment of this application. The following describes steps in FIG. 8.

801. Set a first preset value of duration of a heating mode of a detection and heating material.

Optionally, the control circuit 120 sets the first preset value of duration of the heating mode of the detection and heating material. It should be understood that the target device 200 may alternatively set the first preset value.

802. Switch a working mode of the detection and heating material to a detection mode when heating duration of the detection and heating material is greater than or equal to the first preset value.

Optionally, when the heating duration of the detection and heating material reaches the first preset value, the control circuit 120 instructs the detection and heating circuit 110 to switch the working mode of the detection and heating material to the detection mode.

803. A detection and heating circuit obtains a second bioelectric signal of a user by using the detection and heating material, and processes the obtained second bioelectric signal to obtain third detection information corresponding to the second bioelectric signal.

Optionally, in the detection mode, the detection and heating circuit 110 obtains the second bioelectric signal of the user by using the detection and heating material, and processes the obtained second bioelectric signal to obtain the third detection information corresponding to the second bioelectric signal.

804. Send, to a target device, fourth detection information corresponding to the third detection information.

Optionally, the transceiver circuit 130 sends, to the target device 200, the fourth detection information corresponding to the third detection information, where the fourth detection information may be the third detection information itself, or may be a decomposed signal of the third detection information, or may be a feature signal of a decomposed signal of the third detection information.

805. The target device receives the fourth detection information.

Optionally, the transceiver 210 receives the fourth detection information, and sends the fourth detection information to the processor 220.

806. The target device updates a user state category classification model based on the fourth detection information.

Optionally, the processor 220 receives the fourth detection information sent by the transceiver 210, and updates the user state category classification model based on the fourth detection information.

It should be understood that the first preset value may be a value less than the heating duration of the heating mode, and this is equivalent to pausing heating in a heating process to detect the second bioelectric signal. Alternatively, the first preset value may be a value greater than or equal to the heating duration of the heating mode, and this is equivalent to detecting the second bioelectric signal after the heating ends.

For example, the duration of performing heating is originally set to 10 minutes, the first preset value is 5 minutes, the detection and heating material pauses heating after performing heating for 5 minutes according to a mitigation solution, and works in the detection mode instead, to detect the second bioelectric signal of the user, so that the processor 220 can update the state category classification model based on the second bioelectric signal.

Optionally, when mitigating fatigue of the user, in addition to intervention in the heating mode, auxiliary intervention may be performed through vibration massage, audio playback, or the like. For example, a massage apparatus is added, and when mitigation is performed, auxiliary massage vibration may be performed at a specific position, thereby better mitigating the fatigue of the user.

Mitigating fatigue of a human body by heating is a common way to improve a mental state of a user. When a heating apparatus transmits beneficial heat radiation to the human body, the activity of biomolecules in the human body can be activated, and the human body can feel comfortable and relaxed.

A massage manner is to relax the human body by performing beneficial vibration on muscles or acupoints of the human body, and strength of the massage can be changed by adjusting a frequency and/or an amplitude of the vibration.

Optionally, fatigue categories may be classified into a category 1- non-fatigue, a category 2- general fatigue, and a category 3- great fatigue.

Optionally, similarly to the classification of fatigue categories, the strength of massage vibration may be classified into three strength levels: weak, intermediate, and strong; similarly, the heating temperature is classified into three temperature levels: low, intermediate, and high, and the operation duration is classified into three levels: short, intermediate, and long.

For example, a mitigation solution may be selected by using a mapping relationship between a fatigue category and the mitigation solution shown in Table 2. Different mitigation solutions correspond to different heating temperatures, massage strength, and operation duration. The operation duration refers to the heating duration and the massage duration.

**Table 2**

| Fatigue category | Heating temperature | Massage strength | Operation duration |
| --- | --- | --- | --- |
| Category 1 | 38°C | Weak | 10 minutes |
| Category 2 | 40°C | Intermediate | 15 minutes |
| Category 3 | 42°C | Strong | 25 minutes |

For example, when the fatigue category is the category 1, the heating temperature is set to 38°C, the massage strength is set to "weak", and the operation duration of heating and massage is set to 10 minutes. During working, heating and massage are performed on the user for 10 minutes, the heating temperature is 38°C, and the massage strength is "weak".

It should be understood that Table 2 is merely an example of selecting a mitigation solution based on a fatigue category, and data in Table 2 is also merely an example. However, this is not limited, and other mapping relationships and data settings may also exist. For example, when the fatigue category is the category 1, only the heating temperature is set and no massage is performed; alternatively, the heating temperature is set to 39°C, the massage vibration strength is set to intermediate, and expected complete mitigation duration is set to 8 minutes. For another example, operation duration of heating and operation duration of massage may be different. Details are not described herein.

Optionally, when the fatigue of the user is mitigated in a manner of heating and massage, a control circuit shown in FIG. 9 may be used to perform a user fatigue mitigation solution. With reference to FIG. 9, the following uses the mapping relationship provided in Table 2 as an example for description.

FIG. 9 is a schematic diagram of a structure of an intelligent control apparatus according to an embodiment of this application. As shown in FIG. 9, the apparatus 900 includes a control module 910, a heating/massage module 920, a detection module 930, and a transceiver module 940. It should be understood that each of the foregoing modules in FIG. 9 may be implemented in a form of hardware such as a circuit, or may be implemented in a form of a combination of hardware and software. Details are not described herein.

The control module 910 is configured to control switching of a working mode, that is, configured to control on/off of the heating/massage module 920 and configured to control on/off of the detection module 930, and further configured to control the heating/massage module 920 to perform an improvement solution. The heating/massage module 920 is configured to perform the improvement solution according to an instruction of the control module 910. The detection module 930 is configured to detect a bioelectric signal of a user, and perform certain processing on the detected bioelectric signal. The transceiver module 940 is configured to receive detection information from the detection module 930, and send the detection information to a target device (for example, a cloud device or a terminal); and is further configured to receive, from the target device, indication information corresponding to the detection information, and send the indication information to the control module 910.

It should be noted that, in actual application, the intelligent control apparatus provided in this embodiment of this application may further set an initial mode or a power-on mode. For example, the power-on mode may be set to a detection mode. When the user is using the intelligent control apparatus, detection is performed on the user after power-on, and then a heating temperature and/or heating duration are/is determined based on the detected bioelectric signal. For another example, the initial mode or the power-on mode may alternatively be set to a mode that can be manually selected and switched by the user. For example, a mode selection module may be set, so that the user can autonomously select the initial mode or the power-on mode.

Optionally, the control module 910 may be configured to perform an operation or processing of the control circuit 110 shown in FIG. 1.

Optionally, the detection module 930 may be connected to a detection and heating material provided in this embodiment of this application. When working, the detection and heating material captures a bioelectric signal, and transmits the bioelectric signal to the detection module 930 by using, for example, a wire. The detection module 930 processes the bioelectric signal obtained by the detection and heating material. Optionally, the detection module 930 may use a same structure as the detection circuit 400 shown in FIG. 4.

It should be understood that the detection module 930 may also include the detection and heating material provided in this embodiment of this application, that is, the detection and heating material is used as a component of the detection module 930.

Optionally, the bioelectric signal processed by the detection module 930 may be sent to the transceiver module 940, and then the transceiver module 940 sends the bioelectric signal to the target device.

Optionally, a signal decomposition module may be further set in the detection module 930, to decompose the processed bioelectric signal to obtain a decomposed signal of the bioelectric signal, and send the decomposed signal to the transceiver module 940, and then the transceiver module 940 sends the decomposed signal to the target device.

Further, a feature extraction module may be further set in the detection module 930 to perform feature extraction on the decomposed signal obtained by using the signal decomposition module, to obtain a feature signal of the decomposed signal, and send the feature signal to the transceiver module 940, and then the transceiver module 940 sends the feature signal to the target device.

It should be noted that the foregoing processes such as bioelectric signal processing, signal decomposition, and feature extraction may all use the same method provided in the foregoing related processes. For example, signal decomposition is performed by using an ICA method. Details are not described herein again. It should also be understood that the signal decomposition module and the feature extraction module may be set in the detection module 930, or may be set in the control module 910, or may be set as an independent module. Details are not described herein again.

Optionally, after receiving the detection information sent by the transceiver module, the target device may set, based on the detection information, an appropriate solution for improving a state of the user, that is, set the heating duration, the heating temperature, massage duration, massage strength, and the like, generate indication information corresponding to the foregoing improvement solution, and send the indication information to the transceiver module 940.

After receiving the indication information, the transceiver module 940 sends the indication information to the control module 910. The control module 910 converts, based on the indication information, a corresponding indication into an instruction that can be executed by the heating/massage module 920, for example, on/off of an electrical signal or a magnitude of an electrical signal, to instruct the heating/massage module 920 to perform a corresponding action.

The following illustrates a process of intelligently improving the state of the user by using the apparatus shown in FIG. 9.

Step 1: The detection module 930 obtains a first bioelectric signal of the user in a time period by using the detection and heating material.

Step 2: The detection module 930 processes the first bioelectric signal to obtain first detection information corresponding to the first bioelectric signal.

Step 3: The transceiver module 940 sends, to the target device, second detection information corresponding to the first detection information, where the second detection information may be the first detection information itself, or may be a decomposed signal of the first detection information, or may be a feature signal of a decomposed signal of the first detection information.

Step 4: The target device receives the second detection information, analyzes the second detection information, and determines a state category of the user. The related method provided above may be used to determine the state category of the user.

Step 5: The target device determines an improvement solution based on the state category, and generates indication information used to indicate the improvement solution. For example, the target device determines, by analyzing the second detection information, that a fatigue category of the user in the time period is "category 1", and selects, based on a mapping relationship in Table 2, a mitigation solution in which "the heating temperature is 38°C, massage strength is weak, and operation duration of both heating and massage is 10 minutes"; and accordingly, the target device generates the first instruction information used to indicate the foregoing mitigation solution.

Step 6: The target device sends the first instruction information to the apparatus 900 by using the transceiver module 940.

Step 7: The transceiver module 940 receives the first instruction information, and sends the first instruction information to the control module 910.

Step 8: The control module 910 converts, based on the improvement solution indicated by the indication information, the improvement solution into an instruction that can be executed by the heating/massage module 920, and sends the instruction to the heating/massage module 920, where the instruction may be, for example, on/off of an electrical signal or a magnitude of an electrical signal. The operation duration is controlled by controlling on/off duration of the electrical signal, and the heating temperature and/or vibration strength of the operation are/is controlled by controlling the magnitude of the electrical signal.

Step 9: The heating/massage module 920 performs a corresponding action according to the instruction of the control module 910, to perform the foregoing improvement solution. For example, corresponding to the example in step 4, the user is heated and massaged for 10 minutes, the heating temperature is 38°C, and the massage strength is weak.

It should be noted that, when performing corresponding steps, the foregoing modules may use the foregoing related method. For brevity, details are not described herein again. It should also be understood that the apparatus shown in FIG. 9 may be further used to obtain a user state category classification model, and update the user state category classification model. The related method has been described in FIG. 1. The processes are similar, but execution entities are different. For example, steps performed by the control circuit 110 may be performed by the control module 910. Therefore, details are not described herein again.

Optionally, the heating/massage module 920 may include any detection and heating material provided in this embodiment of this application, for heating.

Optionally, the heating/massage module 920 may include a vibration massage apparatus, and the vibration massage apparatus may be disposed at specific positions for massaging these specific positions. For example, the vibration massage apparatus may be disposed at some acupoints. Optionally, the vibration massage apparatus may also be made of the detection and heating material provided in this embodiment of this application.

It should be understood that, when the heating/massage module 920 includes only a heating module, the detection and heating material provided in this embodiment of this application may be used for implementation. In this case, the heating/massage module 920 and the detection module 930 may be combined into a detection and heating module, and the detection and heating module may have the same functions as the detection and heating circuit 110 shown in FIG. 1.

It should be understood that the heating material and the vibration massage apparatus may be included in the heating/massage module 920, or may be an external apparatus electrically connected to the heating/massage module 920. It should also be understood that another intervention apparatus may be added to the apparatus 900. Details are not described herein.

Optionally, different mitigation solutions may be further set for different positions.

For example, by reading a position of the detection and heating material and the detected bioelectric signal, it is found that there is fatigue at a position A and a position B, where a fatigue category at the position A is "category 2-general fatigue", and a fatigue category at the position B is "category 1- non-fatigue". In this case, different mitigation solutions may be set for the position A and the position B respectively based on the mapping relationship in Table 2. For example, heating and massage operations are performed at the position A based on data in a row 2 of Table 2, while only a heating operation is performed at the position B.

Optionally, the fatigue mitigation solution may be further set to a manner that can be autonomously configured by the user, so that the user can autonomously select different fatigue mitigation solutions. For example, the user may select, by using a visual operation module, a heating temperature, massage strength, and operation duration.

Optionally, an appropriate mitigation solution may be further provided based on an environment in which the user is located and a historical selection. For example, a fatigue mitigation solution may be determined based on an itinerary of the user.

Assuming that after the itinerary of the user is learned by using a terminal device of the user or a cloud device, it is predicted that duration within which the user can use an intelligent wearable device is only 10 minutes, and the fatigue category of the user is "general fatigue", a fatigue mitigation solution corresponding to "great fatigue" may be selected, to mitigate fatigue of the user as soon as possible in a short time.

For another example, the user is currently taking a lunch break, the lunch break is one hour, and the current fatigue category of the user is "great fatigue". In this case, the heating temperature and the massage strength corresponding to "general fatigue" may be selected, but the operation duration is prolonged.

In the foregoing process, determining different mitigation solutions based on different fatigue categories, determining a mitigation solution by comprehensively considering the environment in which the user is located and historical usage, allowing the user to autonomously select the mitigation solution, and the like can provide a more accurate and friendly mitigation solution for the user, thereby improving user experience.

Optionally, a first preset value of duration of a heating mode of the detection and heating material may be further set, and heating mitigation is performed on the user within duration of the first preset value; and after the duration defined by the first preset value, the detection and heating material switches to work in the detection mode, and detects a second bioelectric signal, so that the target device in FIG. 1 and FIG. 9 can update the fatigue category classification model based on third detection information corresponding to the second bioelectric signal or fourth detection information corresponding to third detection information. In addition, the target device may further update the foregoing fatigue mitigation solution based on a current fatigue mitigation status of the user.

It should be understood that the first preset value may be a value less than the heating duration of the heating mode, and this is equivalent to pausing heating in a heating process to detect the second bioelectric signal. Alternatively, the first preset value may be a value greater than or equal to the heating duration of the heating mode, and this is equivalent to detecting the second bioelectric signal after the heating ends.

For example, the duration of performing heating is originally set to 10 minutes, the first preset value is 5 minutes, the detection and heating material pauses heating after performing heating for 5 minutes according to the mitigation solution, and works in the detection mode instead, to detect the second bioelectric signal of the user, so that the target device can update the fatigue category classification model based on the second bioelectric signal.

It should be noted that the second bioelectric signal refers to a bioelectric signal detected after the heating mode is performed for certain duration. The second bioelectric signal is used to update the fatigue category classification model, and is different from the first bioelectric signal and a third bioelectric signal in terms of a detection time and usage, but can also be obtained through detection by the detection and heating material.

FIG. 10 is a schematic diagram of a hardware structure of an intelligent control apparatus according to an embodiment of this application. As shown in FIG. 10, the intelligent control apparatus includes a detection and heating material 1001, a power supply 1002, a heating circuit 1003, a temperature controller 1004, an analog switch group 1005, a control logic transmitter 1006, and a bioelectric processor 1007, and may further include a communication transmitter 1008 and a communication receiver 1009.

The detection and heating material 1001 may be any one of the foregoing detection and heating materials for detecting a bioelectric signal in a detection mode and heating in a heating mode.

The power supply 1002 is configured to provide a current or voltage signal for the apparatus.

The heating circuit 1003 is configured to control a heating temperature and heating duration of the detection and heating material 1001 under control of the temperature controller 1004. The heating circuit 1003 may be the heating circuit shown in FIG. 2 or FIG. 3.

The temperature controller 1004 may be the temperature controller described above, and is configured to control the heating temperature of the detection and heating material within a range.

The analog switch group 1005 is configured to control connection/disconnection of a circuit path connected to the detection and heating material 1001, to implement switching of a working mode of the detection and heating material 1001.

The control logic transmitter 1006 is configured to send an instruction to the temperature controller 1004 to instruct the temperature controller 1004 to perform heating to a corresponding temperature.

The bioelectric processor 1007 is configured to process the bioelectric signal (analog bioelectric signal) obtained by using the detection and heating material 1001.

The communication transmitter 1008 is configured to send the bioelectric signal processed by the bioelectric processor 1007.

The communication receiver 1009 is configured to receive indication information used to indicate the heating temperature and/or the heating duration.

The apparatus shown in FIG. 10 may be an implementation of the apparatus shown in FIG. 1. The detection and heating circuit 110 shown in FIG. 1 may include the detection and heating material 1001, the heating circuit 1003, and the bioelectric processor 1007. The transceiver circuit 130 shown in FIG. 1 may include the communication transmitter 1008 and the communication receiver 1009. The control circuit 120 shown in FIG. 1 may include the temperature controller 1004, the analog switch group 1005, and the control logic transmitter 1006.

FIG. 11 is a schematic diagram of an intelligent control apparatus according to an embodiment of this application. The intelligent control apparatus shown in FIG. 11 may be used, for example, for an intelligent wearable device. As shown in FIG. 11, the intelligent control apparatus 100' includes a detection and heating circuit 110' and a control circuit 120'.

Optionally, the detection and heating circuit 110' may use a same structure as the detection and heating circuit 110 shown in FIG. 1, and may perform a same operation as the detection and heating circuit 110 shown in FIG. 1, to implement a same function, and the detection and heating circuit 110' may also include the detection circuit and/or the heating circuit or the like shown in FIG. 2 and FIG. 3. Details are not described herein again.

Optionally, the control circuit 120' may use a same structure as the control circuit 120 shown in FIG. 1, and/or may perform a same operation as the control circuit shown in FIG. 1, to implement a same function. Details are not described herein again. It should be noted that, in addition to the foregoing operations of the control circuit 120, the control circuit 120' may further perform operations of the processor 220 in the target device 200 shown in FIG. 1, and can implement any same function of the control circuit 120.

It should be understood that the intelligent control apparatus 100' shown in FIG. 11 may implement any function of the intelligent control apparatus 100 shown in FIG. 1, or may implement any function of the processor 220 in the target device 200 shown in FIG. 1. However, because all execution modules or units are in the same apparatus, and interaction similar to communication between the intelligent control apparatus 100 and the target device 200 or the like does not need to be performed, the transceiver circuit 130 and the transceiver circuit 210 shown in FIG. 1 may be omitted. However, addition of the two modules in FIG. 11 does not affect function implementation of the intelligent control apparatus.

The following describes the structure and function of the control circuit 120' by using an example. For some omitted content, refer to the foregoing related descriptions.

Optionally, the control circuit 120' may include a mode control module 121', a processing module 122', an analysis module 123', and a policy module 124'. It should be understood that the foregoing division is division performed based on functional logic, and another division manner may also exist. It should also be understood that each of the foregoing modules may be implemented in a form of hardware such as a circuit, or may be implemented in a form of a combination of hardware and software. Details are not described herein again.

Optionally, the mode control module 121' may have a same function as the control circuit 120 shown in FIG. 1 and/or the control circuit 910 shown in FIG. 9. In other words, the mode control module 121' may be configured to control a working mode of a detection and heating material, and instruct the detection and heating circuit 110' to adjust a parameter in the working mode of the detection and heating material.

Optionally, the processing module 122' may be configured to obtain and process a bioelectric signal or a processed signal of a bioelectric signal from the detection and heating circuit 110' in a detection mode.

In an implementation, the detection and heating circuit 110' obtains a first bioelectric signal of a user, processes the first bioelectric signal, and sends the processed first bioelectric signal (for example, the processed first bioelectric signal may be equivalent to the first detection information described above) to the processing module 122'. The processing module 122' obtains the processed first bioelectric signal, decomposes the processed first bioelectric signal to obtain a decomposed signal of the first bioelectric signal, and then performs feature extraction on the decomposed signal to obtain a feature signal of the decomposed signal.

It should be noted that the foregoing process of signal decomposition and signal feature extraction may alternatively be performed by the detection and heating circuit 110'.

Optionally, the analysis module 123' may perform operations such as determining a state category of the user, training a state category classification model, and updating a user state category classification model in the foregoing processor 220.

Optionally, the policy module 124' may perform the foregoing process of determining a corresponding improvement solution based on the state category of the user in the processor 220.

It should be noted that, in actual application, the intelligent control apparatus provided in this embodiment of this application may further set an initial mode or a power-on mode. For example, the power-on mode may be set to the detection mode. When the user is using the intelligent control apparatus, detection is performed on the user after power-on, and then a heating temperature and/or heating duration are/is determined based on the detected bioelectric signal. For another example, the initial mode or the power-on mode may alternatively be set to a mode that can be manually selected and switched by the user. For example, a mode selection module may be set, so that the user can autonomously select the initial mode or the power-on mode.

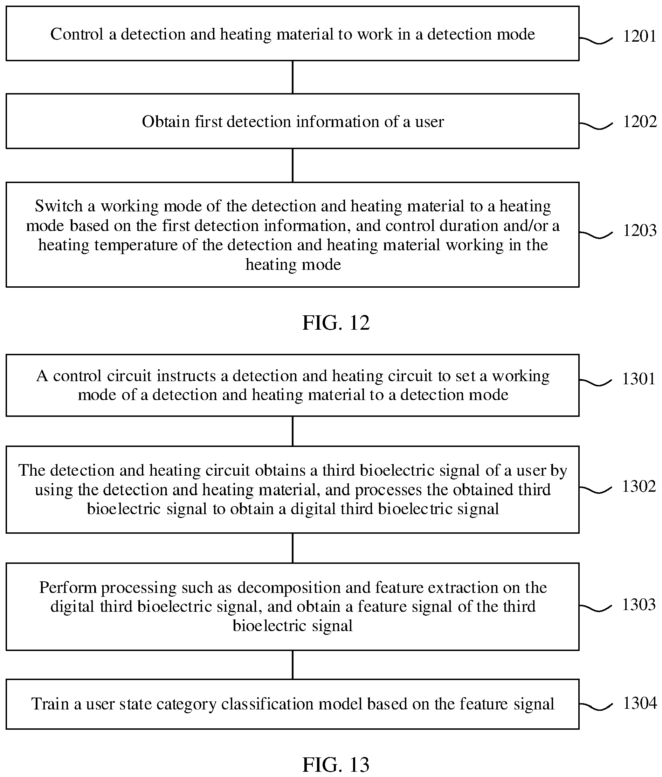

With reference to FIG. 12, the following illustrates a process of intelligently improving a state of a user by using the apparatus shown in FIG. 11. FIG. 12 is a schematic flowchart of a control method for an intelligent control apparatus according to an embodiment of this application. The following describes steps shown in FIG. 12.

1201. Control a detection and heating material to work in a detection mode.

It should be noted that working modes of the detection and heating material include a heating mode and the detection mode, where the detection and heating material obtains a bioelectric signal of a user when working in the detection mode, and performs heating when working in the heating mode.

Optionally, the control module 121' instructs the detection and heating circuit 110' to set the working mode of the detection and heating material to the detection mode.

1202. Obtain first detection information of the user.

Optionally, in the detection mode, the detection and heating circuit 110' obtains the first bioelectric signal of the user by using the detection and heating material, and processes the obtained first bioelectric signal to obtain a digital first bioelectric signal (for example, the first bioelectric signal processed by using the foregoing detection circuit 400). The detection and heating circuit 110' transmits the digital first bioelectric signal to the processing module 122' by using a wire or a data sending mode. The processing module 122' performs processing such as decomposition and feature extraction on the digital first bioelectric signal, and sends an obtained feature signal to the analysis module 123'.

It should be noted that the first detection information may be the digital first bioelectric signal, or may be a feature signal of the first bioelectric signal.

1203. Switch the working mode of the detection and heating material to the heating mode based on the first detection information, and control duration and/or a heating temperature of the detection and heating material working in the heating mode.

Optionally, step 1203 may be performed by using the following method.

1203a. The analysis module 123' receives the feature signal, determines a state category of the user based on the feature signal, and sends the state category of the user to the policy module 124'.

1203b. The policy module 124' obtains the state category, determines a corresponding improvement solution based on the state category, and sends the improvement solution to the mode control module 121'.

Step 7: The mode control module 121' converts the improvement solution into an instruction that can indicate the solution, instructs the detection and heating circuit 110' to switch the working mode of the detection and heating material, so that the detection and heating material works in the heating mode instead, and instructs the detection and heating circuit 110' to control the heating duration and/or the heating temperature of the detection and heating material, so that the detection and heating circuit 110' performs the improvement solution.

1203c. The detection and heating circuit 110' switches the working mode of the detection and heating material to the heating mode according to the instruction of the mode control module 121'.

1203d. In the heating mode, the detection and heating circuit 110' controls the heating duration and/or the heating temperature of the detection and heating material according to the instruction of the mode control module 121', to perform the improvement solution.

It should be noted that the foregoing steps may be performed by using the corresponding method provided above, and the foregoing steps may not be completely performed. For example, the mode control module 121' may only set two working modes of the detection and heating material to perform detection and heating alternately based on fixed duration, and instruct the detection and heating material 110' to perform corresponding actions. In this case, a process such as determining the state category of the user is not required. This example has been described above, and details are not described herein again.

With reference to FIG. 13, the following illustrates a process of obtaining a user state category classification model by using the apparatus shown in FIG. 11. FIG. 13 is a schematic flowchart of a method for obtaining a user state category classification model according to an embodiment of this application. The following describes steps shown in FIG. 13.

1301. A control circuit instructs a detection and heating circuit to set a working mode of a detection and heating material to a detection mode.

Optionally, the control module 121' instructs the detection and heating circuit 110' to set the working mode of the detection and heating material to the detection mode.

1302. The detection and heating circuit obtains a third bioelectric signal of a user by using the detection and heating material, and processes the obtained third bioelectric signal to obtain a digital third bioelectric signal.

Optionally, in the detection mode, the detection and heating circuit 110' obtains the third bioelectric signal of the user by using the detection and heating material, and processes the obtained third bioelectric signal to obtain the digital third bioelectric signal (for example, the third bioelectric signal processed by using the foregoing detection circuit 400).

Optionally, the detection and heating circuit 110' transmits the digital third bioelectric signal to the processing module 122' by using a wire or a data sending mode.

1303. Perform processing such as decomposition and feature extraction on the digital third bioelectric signal, and obtain a feature signal of the third bioelectric signal.

Optionally, the processing module 122' performs processing such as decomposition and feature extraction on the digital third bioelectric signal, and sends the obtained feature signal to the analysis module 123'.

1304. Train a user state category classification model based on the feature signal.

Optionally, the analysis module 123' receives the feature signal, and trains the user state category classification model based on the feature signal.

Optionally, the machine learning method described above may be used to train the user state category classification model by using the obtained third bioelectric signal or the feature signal of the obtained third bioelectric signal.

It should be noted that the state category classification model may also be updated by using the method provided above.

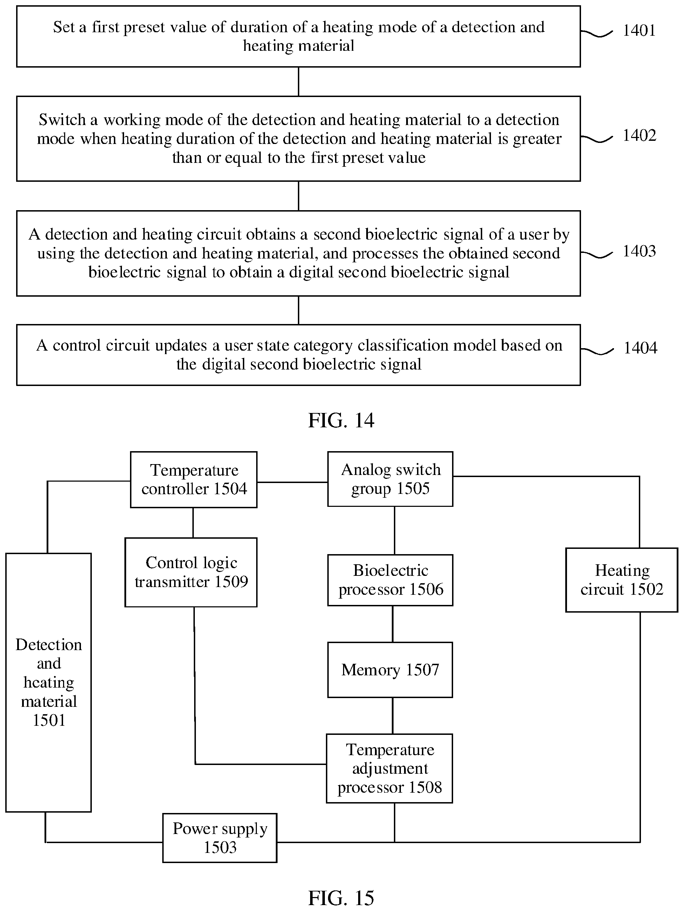

With reference to FIG. 14, the following illustrates a process of updating a user state category classification model by using the apparatus shown in FIG. 11. FIG. 14 is a schematic flowchart of a method for updating a user state category classification model according to an embodiment of this application. The following describes steps shown in FIG. 14.

1401. Set a first preset value of duration of a heating mode of a detection and heating material.

Optionally, the control module 121' may set the first preset value of duration of the heating mode of the detection and heating material.

1402. Switch a working mode of the detection and heating material to a detection mode when heating duration of the detection and heating material is greater than or equal to the first preset value.

Optionally, when the heating duration of the detection and heating material reaches the first preset value, the control module 121' instructs the detection and heating circuit 110' to switch the working mode of the detection and heating material to the detection mode.

1403. A detection and heating circuit obtains a second bioelectric signal of a user by using the detection and heating material, and processes the obtained second bioelectric signal to obtain a digital second bioelectric signal.

Optionally, in the detection mode, the detection and heating circuit 110' obtains the second bioelectric signal of the user by using the detection and heating material, and processes the obtained second bioelectric signal to obtain the digital second bioelectric signal (for example, the second bioelectric signal processed by using the foregoing detection circuit 400).

1404. A control circuit updates a user state category classification model based on the digital second bioelectric signal.

Optionally, assuming that the control circuit includes the modules shown in FIG. 11, the following steps may be used as an implementation process of step 1404.

1404a. The detection and heating circuit 110' transmits the digital second bioelectric signal to the processing module 122' by using a wire or a data sending mode.

1404b. The processing module 122' performs processing such as decomposition and feature extraction on the digital second bioelectric signal, and sends an obtained feature signal to the analysis module 123'.

1404c. The analysis module 123' receives the feature signal, and updates the user state category classification model based on the feature signal.

It should be understood that the first preset value may be a value less than the heating duration of the heating mode, and this is equivalent to pausing heating in a heating process to detect the second bioelectric signal. Alternatively, the first preset value may be a value greater than or equal to the heating duration of the heating mode, and this is equivalent to detecting the second bioelectric signal after the heating ends.

For example, the duration of performing heating is originally set to 10 minutes, the first preset value is 5 minutes, the detection and heating material pauses heating after performing heating for 5 minutes according to a mitigation solution, and works in the detection mode instead, to detect the second bioelectric signal of the user, so that the analysis module 123' can update the fatigue category classification model based on the second bioelectric signal.

It should be noted that, in the apparatus shown in FIG. 11, the vibration massage apparatus described above may also be disposed, to implement an improvement solution combining heating and vibration massage. Details are not described herein again.

FIG. 15 is a schematic diagram of a hardware structure of an intelligent control apparatus according to an embodiment of this application. As shown in FIG. 15, the apparatus includes a detection and heating material 1501, a heating circuit 1502, a power supply 1503, a temperature controller 1504, an analog switch group 1505, a bioelectric processor 1506, a memory 1507, a temperature adjustment processor 1508, and a control logic transmitter 1509. The following describes each part. The power supply 1503 is configured to provide a current or voltage signal for the apparatus.

The detection and heating material 1501 may be any one of the foregoing detection and heating materials for detecting a bioelectric signal in a detection mode and heating in a heating mode.

The heating circuit 1502 is configured to control a heating temperature and heating duration of the detection and heating material 1501 under control of the temperature controller 1504. The heating circuit 1502 may be the heating circuit shown in FIG. 2 or FIG. 3.

The temperature controller 1504 may be the temperature controller described above, and is configured to control the heating temperature of the detection and heating material within a range.

The analog switch group 1505 is configured to control connection/disconnection of a circuit path connected to the detection and heating material 1501, to implement switching of a working mode of the detection and heating material 1501.

The bioelectric processor 1506 is configured to perform processing, such as amplification, filtering, and analog-to-digital conversion, on an analog bioelectric signal obtained by the detection and heating material 1501, to obtain a digital bioelectric signal.

The memory 1507 is configured to store data that needs to be stored in a process, for example, a bioelectric signal or a state category classification model. The processor 1507 may be disposed independently, or may be integrated with another part.

The temperature adjustment processor 1508 is configured to determine the heating temperature and/or the heating duration based on the digital bioelectric signal, and send the heating temperature and/or the heating duration that need/needs to be set, to the temperature controller 1504 by using the control logic transmitter 1509.

The control logic transmitter 1509 is configured to send an instruction from the temperature adjustment processor 1508 to the temperature controller 1504.

The apparatus shown in FIG. 15 may be an implementation of the apparatus shown in FIG. 11. The detection and heating circuit 110' shown in FIG. 11 may include the detection and heating material 1501, the heating circuit 1502, and the bioelectric processor 1506, where the bioelectric processor 1506 is equivalent to the foregoing detection circuit. The control circuit 120' shown in FIG. 11 may include the temperature controller 1504, the analog switch group 1505, the bioelectric processor 1506, the temperature adjustment processor 1508, and the control logic transmitter 1509, and the control circuit 120' may further include the memory 1507.

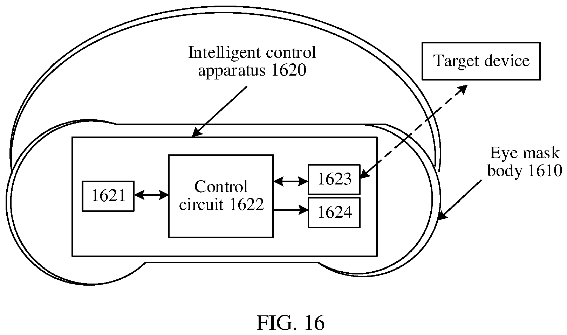

FIG. 16 is a schematic diagram of an intelligent eye mask according to an embodiment of this application. As shown in FIG. 16, the intelligent eye mask includes an eye mask body 1610 and an intelligent control apparatus 1620.

Optionally, the intelligent control apparatus 1620 is any one of the foregoing intelligent control apparatuses. For brevity, details are not described again.

It should be understood that the intelligent control apparatus 1620 may be disposed in an interlayer of the eye mask body 1610, or a detection and heating material of the intelligent control apparatus 1620 may be used as a part of the eye mask body 1610. For example, the detection and heating material is directly woven on a surface of the eye mask body 1610 in a weaving manner.

In an implementation, the intelligent control apparatus 1620 includes a detection and heating circuit 1621 and a control circuit 1622. During working, the detection and heating circuit 1621 is configured to detect a bioelectric signal and transmit the bioelectric signal to the control circuit 1622; the control circuit 1622 processes and decomposes the bioelectric signal to obtain, for example, an electroencephalogram signal of a head, an electro-oculogram signal of an eye, and an electromyography signal of a face, determines a state category of a user based on the signals obtained through decomposition, generates a corresponding state mitigation solution, and controls the detection and heating material 1621 to perform heating to mitigate a brain and/or eye state of the user.

It should be noted that, alternatively, the foregoing processing and decomposition of the bioelectric signal and feature extraction may be partially or completely performed by the detection and heating circuit 1621, that is, what the detection and heating circuit 1621 transmits to the control circuit 1623 may be a preprocessed bioelectric signal (for example, the digital bioelectric signal described above), or may be a decomposed signal of the bioelectric signal, or may be a feature signal of the bioelectric signal. Details are not described herein again.

Optionally, the detection and heating material in the detection and heating circuit 1621 is made of a material having both conductivity and heat conductivity, such as silver plating or graphene fiber, and has two working modes: a detection mode (used to capture a bioelectric signal in the detection mode) and a heating mode (used for heating in the heating mode).

Optionally, the intelligent control apparatus 1620 may control, based on the bioelectric signal detected by the detection and heating circuit 1621 and/or based on the state category, heating duration and/or a heating temperature of the detection and heating material working in the heating mode. This is equivalent to autonomously controlling parameters of the heating mode by the intelligent control apparatus 1620 based on the detected bioelectric signal, or autonomously determining the state category of the user based on the detected bioelectric signal, and controlling, based on the state category of the user, the heating duration and/or the heating temperature of the detection and heating material working in the heating mode.

In another implementation, the intelligent control apparatus 1620 includes a detection and heating circuit 1621, a control circuit 1622, and a transceiver circuit 1623. The transceiver circuit 1623 is configured to perform data or signal transmission such as communication with a target device (for example, a terminal device or a cloud device), and specifically send a bioelectric signal or a feature signal of a bioelectric signal to the target device, and receive indication information of a state category or indication information of an improvement solution sent by the target device. The transceiver circuit 1623 may be integrated in the control circuit 1622 or may be independent of the control circuit 1622.

The detection and heating circuit 1621 is configured to detect the bioelectric signal and perform certain processing on the bioelectric signal, and may perform only preprocessing procedures such as amplification, filtering, and analog-to-digital conversion, or may perform some or all other processing such as decomposition and feature extraction. In other words, detection information obtained by the detection and heating circuit 1621 and corresponding to the bioelectric signal may be any one of a digital bioelectric signal, a non-decomposed bioelectric signal, or a feature signal of the bioelectric signal.

The transceiver circuit 1623 processes the detection information obtained from the detection and heating circuit 1621 and sends the processed detection information to the target device, or sends the detection information to the target device without processing. In other words, the detection information sent by the transceiver circuit 1623 to the target device may be the same as any one of the digital bioelectric signal, the non-decomposed signal of the bioelectric signal, or the feature signal of the bioelectric signal.

It should be understood that, after receiving the detection information from the transceiver circuit 1623, the target device determines the state category of the user based on the detection information, and generates a corresponding state improvement solution. The state improvement solution can be used to indicate and control heating duration and/or a heating temperature of the detection and heating material 1621 to mitigate a brain and/or eye state of the user.

The transceiver circuit 1623 may receive the indication information of the state category sent by the target device, and send the indication information to the control circuit 1622, so that the control circuit 1622 can obtain the state category based on the indication information and determine a mitigation solution based on the state category, to control the heating duration and/or the heating temperature of the detection and heating material working in the heating mode. This is equivalent to determining the state category of the user by the target device, and determining an improvement solution based on the state category and performing the improvement solution by the intelligent control apparatus. Alternatively, the transceiver circuit 1623 may receive indication information of an improvement solution sent by the target device, and send the indication information to the control circuit 1622, so that the control circuit 1622 can instruct, based on the indication information, the detection and heating circuit 1621 to perform the improvement solution. This is equivalent to determining the state category of the user and determining the improvement solution by the target device.

Optionally, the intelligent control apparatus 1620 may further include a massage vibration intervention apparatus 1624, configured to generate a vibration, and implement a function of massage for the user. The massage vibration intervention apparatus 1624 may be disposed at a same position as the detection and heating material, so that when a bioelectric signal abnormality is detected at the position, the massage vibration can be performed for the position, thereby providing a more personalized mitigation service.

It should be noted that some content of the description of the intelligent control apparatus 1620 and the description of the foregoing intelligent control apparatus may correspond to each other. Therefore, for a part not described, refer to the content of the foregoing intelligent control apparatus.

The intelligent control apparatus provided in this embodiment of this application may be further applied to another wearable device, for example, may be applied to a smart scarf, smart glasses, or a smart belt.

Optionally, the intelligent wearable device may include any one of the foregoing intelligent control apparatuses, and the intelligent wearable device may further include a wearable device body. Optionally, the detection and heating material of the intelligent control apparatus may be a component of the wearable device body.

Optionally, any one of the foregoing intelligent control apparatuses may be applied to a virtual reality (virtual reality, VR) device, for example, VR glasses, to intelligently mitigate user fatigue, for example, eye fatigue.

For further example, the intelligent control apparatus shown in FIG. 1 or FIG. 10 and an intelligent analysis apparatus are disposed in the VR glasses. The intelligent control apparatus includes a detection and heating circuit, a transceiver circuit, and a control circuit. The control circuit is configured to control a detection and heating material to detect a bioelectric signal or perform heating. The intelligent analysis apparatus is configured to determine a state category of a user based on received detection information (the bioelectric signal, a feature signal of the bioelectric signal, or the like), and/or configured to determine an improvement solution based on a fatigue category. This is equivalent to being configured to perform an operation of the foregoing target device. When working, the detection and heating material detects the bioelectric signal of the user, and sends, to the intelligent analysis apparatus by using the transceiver circuit, the detection information corresponding to the bioelectric signal. The intelligent analysis apparatus determines the fatigue category of the user based on the signal feature of the received bioelectric signal, and sends indication information about the fatigue category of the user or the improvement solution to the control circuit by using the transceiver circuit, so that the control circuit can control heating duration and/or a heating temperature of the detection and heating material accordingly.

For another example, the intelligent control apparatus shown in FIG. 11 or FIG. 15 is disposed in the VR glasses. The intelligent control apparatus includes a detection and heating circuit and a control circuit. The detection and heating circuit includes a detection and heating material for detecting a bioelectric signal and performing certain processing on the bioelectric signal. The control circuit is configured to perform an operation such as determining an improvement solution based on the bioelectric signal. For a specific process, refer to the related description above. For brevity, details are not described again.

Further, to make services of the VR glasses more personalized, vibration massage patches may be further disposed near eye acupoints, massage strength and/or duration of the vibration massage patches are/is controlled based on the fatigue category of the user, and different heating duration and/or different heating temperatures may be set for different positions.

Optionally, any one of the foregoing intelligent control apparatuses may be further applied to a smart belt for mitigating lower back pain, and the heating duration and/or the heating temperature of the detection and heating material may be controlled when an abnormality is determined based on the bioelectric signal detected by the detection and heating material, to mitigate the lower back pain or fatigue.

Further, to make services of the wearable device more personalized, intervention in a manner of heating and massage or the like can be further focused on different abnormal positions. For example, in the smart belt for mitigating low back pain, if the detection and heating material detects an abnormal bioelectric signal at a position of a lumbar vertebra, auxiliary vibration massage may be performed at the position during heating.

In addition, any one of the foregoing intelligent control apparatuses may be further applied to another wearable device such as a head-mounted massage device. Details are not described herein again.

In the foregoing example, the intelligent control apparatus provided in this embodiment of this application is applied to the wearable device to implement a function of intelligently improving a state of the user, and the detection and heating material is applied to reduce structural complexity of the wearable device and improve wear comfort of the user. In addition, in the foregoing example, intervention in a manner of heating and massage or the like can be further focused on different abnormal positions, so that the services of the wearable device are more personalized.

In addition, this application further provides a computer-readable storage medium. The computer-readable storage medium stores computer instructions. When the computer instructions are run on a computer, the computer is enabled to perform operations and/or processing in the control method for the intelligent control apparatus provided in this application.

This application further provides a computer program product. The computer program product includes computer program code. When the computer program code is run on a computer, the computer is enabled to perform operations and/or processing in the control method for the intelligent control apparatus provided in this application.

A person of ordinary skill in the art may be aware that, various illustrative logical blocks (illustrative logical block) and steps (step) that are described with reference to embodiments disclosed in this specification can be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief descriptions, for a detailed working process of the foregoing systems, apparatuses, and units, refer to a corresponding process in the foregoing method embodiments. Details are not described herein again.

In the several embodiments provided in this application, it should be understood that the disclosed systems, apparatuses, and methods may be implemented in other manners. For example, the foregoing apparatus embodiments are merely examples. For example, division of the units is merely logical function division and may be other division during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in an electronic, a mechanical, or another form.

The units described as separate parts may or may not be physically separate, and parts displayed as units may or may not be physical units, that is, may be located in one position, or may be distributed on a plurality of network units. Some or all of the units may be selected based on actual requirements to achieve the objectives of the solutions in the embodiments.

In addition, functional units in the embodiments of this application may be integrated into one unit, or each of the units may exist alone physically, or two or more units are integrated into one unit.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1. An intelligent control apparatus, comprising a control circuit, a transceiver circuit, and a detection and heating circuit;

   the detection and heating circuit comprises a detection and heating material;
   the detection and heating circuit is configured to control, according to an instruction of the control circuit, switching of a working mode of the detection and heating material, wherein the working mode of the detection and heating material comprises a detection mode and a heating mode;
   when the working mode of the detection and heating material is the detection mode, the detection and heating circuit is configured to obtain first detection information of a user by using the detection and heating material, wherein the first detection information is obtained based on a first bioelectric signal of the user;
   the transceiver circuit is configured to send second detection information to a target device, wherein the second detection information is obtained based on the first detection information;
   the transceiver circuit is further configured to receive first instruction information sent by the target device and corresponding to the second detection information, and send the first instruction information to the control circuit; and
   the control circuit is configured to instruct, according to the first instruction information, the detection and heating circuit to switch the working mode of the detection and heating material to the heating mode, and instruct, according to the first instruction information, the detection and heating circuit to control heating duration and/or a heating temperature of the detection and heating material in the heating mode.

2. The apparatus according to claim 1, wherein the detection and heating circuit is specifically configured to: when the detection and heating material is controlled to work in the detection mode, block an electrical signal that is applied to the detection and heating material and used to control heating of the detection and heating material; or when the detection and heating material works in the heating mode, control the heating temperature of the detection and heating material by controlling an electrical signal applied to the detection and heating material.

3. The apparatus according to claim 1 or 2, wherein the second detection information is the same as the first detection information.

4. The apparatus according to claim 1 or 2, wherein the control circuit is further configured to: obtain the first detection information, and decompose the first detection information, to obtain the second detection information.

5. The apparatus according to claim 1 or 2, wherein the control circuit is further configured to: obtain the first detection information, decompose the first detection information, to obtain a decomposed signal of the first detection information, and perform feature extraction on the decomposed signal to obtain the second detection information.

6. The apparatus according to any one of claims 1 to 5, wherein the control circuit is further configured to: when the duration of the detection and heating material working in the heating mode is greater than or equal to a first preset value, instruct the detection and heating circuit to switch the working mode of the detection and heating material to the detection mode;

   the detection and heating circuit is further configured to obtain third detection information of the user by using the detection and heating material, wherein the third detection information is obtained based on a second bioelectric signal of the user; and
   the transceiver circuit sends fourth detection information to the target device, wherein the fourth detection information is obtained based on the third detection information.

7. An intelligent control apparatus, comprising a control circuit and a detection and heating circuit;

   the detection and heating circuit comprises a detection and heating material;
   the detection and heating circuit is configured to control, according to an instruction of the control circuit, switching of a working mode of the detection and heating material, wherein the working mode of the detection and heating material comprises a detection mode and a heating mode;

the detection and heating circuit is configured to obtain first detection information of a user by using the detection and heating material in the detection mode, wherein the first detection information is obtained based on a first bioelectric signal of the user; and

the control circuit is configured to instruct, based on the first detection information, the detection and heating circuit to switch the working mode of the detection and heating material to the heating mode, and instruct the detection and heating circuit to control heating duration and/or a heating temperature of the detection and heating material working in the heating mode.

8. The apparatus according to claim 7, wherein the detection and heating circuit is specifically configured to: when the detection and heating material works in the detection mode, block an electrical signal that is applied to the detection and heating material and used to control heating of the detection and heating material; or when the detection and heating material works in the heating mode, control the heating temperature of the detection and heating material by controlling an electrical signal applied to the detection and heating material.

9. The apparatus according to claim 7 or 8, wherein the control circuit is specifically configured to: determine a state category of the user based on the first detection information, and determine an improvement solution corresponding to the state category, wherein the improvement solution includes the duration and/or the heating temperature of the detection and heating material working in the heating mode;

the control circuit is further configured to instruct the detection and heating circuit to switch the working mode of the detection and heating material to the heating mode, and instruct the detection and heating circuit to perform the improvement solution; and

the state category is used to indicate a state of the user, and the state of the user includes at least one of a fatigue state, an anxiety state, a sleep state, a tension state, and a depression state.

10. The apparatus according to claim 9, wherein the control circuit is specifically configured to: decompose the first detection information to obtain a decomposed signal of the first detection information;

perform feature extraction on the decomposed signal to obtain a feature signal of the decomposed signal; and determine the state category of the user based on the feature signal and a state category classification model.

11. The apparatus according to claim 10, wherein the control circuit is further configured to: when the duration of the detection and heating material working in the heating mode is greater than or equal to a first preset value, instruct the detection and heating circuit to switch the working mode of the detection and heating material to the detection mode;

the detection and heating circuit is further configured to obtain second detection information of the user by using the detection and heating material, wherein the second detection information is obtained based on a second bioelectric signal of the user; and

the control circuit is further configured to update the state category classification model based on the second detection information.

12. An intelligent system, wherein the intelligent system comprises at least one intelligent control apparatus according to any one of claims 1 to 6 and a target device;

the target device is configured to: receive second detection information from the intelligent control apparatus; determine a state category of a user based on the second detection information; determine first instruction information based on the state category; and send the first instruction information to the intelligent control apparatus, wherein the first instruction information is used to instruct a detection and heating material in the intelligent control apparatus to switch to a heating mode, and indicate duration and/or a heating temperature of the detection and heating material working in the heating mode; and

the state category is used to indicate a state of the user, and the state of the user includes at least one of a fatigue state, an anxiety state, a sleep state, a tension state, and a depression state.

13. The system according to claim 12, wherein the target device is specifically configured to determine the state category of the user based on the second detection information and a user state category classification model.

**14.** The system according to claim 13, wherein the target device is further configured to update the state category classification model based on fourth detection information from the intelligent control apparatus.

**15.** A control method for a detecting and heating material, wherein the method comprises:

controlling the detection and heating material to work in a detection mode, wherein a working mode of the detection and heating material includes a heating mode and the detection mode, and the detection and heating material obtains a bioelectric signal of a user when working in the detection mode and performs heating when working in the heating mode;
obtaining first detection information of the user, wherein the first detection information is obtained based on a first bioelectric signal of the user;
sending, to a target device, second detection information, wherein the second detection information is obtained based on the first detection information;
receiving first instruction information sent by the target device and corresponding to the second detection information; and
switching the working mode of the detection and heating material to the heating mode according to the first instruction information, and controlling duration and/or a heating temperature of the detection and heating material working in the heating mode.

**16.** The method according to claim 15, wherein the controlling the detection and heating material to work in a detection mode comprises:

blocking an electrical signal that is applied to the detection and heating material and used to control heating of the detection and heating material; and
the controlling a heating temperature of the detection and heating material working in the heating mode comprises:
controlling the heating temperature of the detection and heating material by controlling an electrical signal applied to the detection and heating material.

**17.** The method according to claim 15 or 16, wherein the second detection information is the same as the first detection information.

**18.** The method according to claim 15 or 16, wherein the method further comprises: decomposing the first detection information, to obtain the second detection information.

**19.** The method according to claim 15 or 16, wherein the method further comprises:

decomposing the first detection information, to obtain a decomposed signal of the first detection information; and
performing feature extraction on the decomposed signal to obtain the second detection information.

**20.** The method according to any one of claims 15 to 19, wherein the method further comprises:

when the duration of the detection and heating material working in the heating mode is greater than or equal to a first preset value, switching the working mode of the detection and heating material to the detection mode;
obtaining third detection information of the user, wherein the third detection information is obtained based on a second bioelectric signal of the user; and
sending fourth detection information to the target device, wherein the fourth detection information is obtained based on the third detection information.

**21.** A control method for a detecting and heating material, wherein the method comprises:

controlling the detection and heating material to work in a detection mode, wherein a working mode of the detection and heating material includes a heating mode and the detection mode, and the detection and heating material obtains a bioelectric signal of a user when working in the detection mode and performs heating when working in the heating mode;
obtaining first detection information of the user, wherein the first detection information is obtained based on a first bioelectric signal of the user; and
switching the working mode of the detection and heating material to the heating mode based on the first detection information, and controlling duration and/or a heating temperature of the detection and heating material working in the heating mode.

22. The method according to claim 21, wherein the controlling the detection and heating material to work in a detection mode comprises:

blocking an electrical signal that is applied to the detection and heating material and used to control heating of the detection and heating material; and
the controlling a heating temperature of the detection and heating material working in the heating mode comprises:
controlling the heating temperature of the detection and heating material by controlling an electrical signal applied to the detection and heating material.

23. The method according to claim 21 or 22, where the switching the working mode of the detection and heating material to the heating mode based on the first detection information, and controlling duration and/or a heating temperature of the detection and heating material working in the heating mode comprises:

determining a state category of the user based on the first detection information, wherein a state of the user includes at least one of a fatigue state, an anxiety state, a sleep state, a tension state, and a depression state;
determining an improvement solution based on the state category of the user, wherein the improvement solution comprises detecting the duration and/or the heating temperature of the detection and heating material working in the heating mode;
switching the working mode of the detecting and heating material to the heating mode; and
performing the improvement solution.

24. The method according to claim 23, wherein the determining a state category of the user based on the first detection information comprises:

decomposing the first detection information, to obtain a decomposed signal of the first detection information; and
perform feature extraction on the decomposed signal to obtain a feature signal of the decomposed signal; and
determine the state category of the user based on the feature signal and a state category classification model.

25. The method according to claim 24, wherein the method further comprises:

when the duration of the detection and heating material working in the heating mode is greater than or equal to a first preset value, switching the working mode of the detection and heating material to the detection mode;
obtaining second detection information of the user, wherein the second detection information is obtained based on a second bioelectric signal of the user; and
updating the state category classification model based on the second detection information.

26. A computer storage medium, wherein the computer storage medium stores a computer program, and when the computer program is executed by a computer, the method according to any one of claims 15 to 20 is implemented.

27. A computer storage medium, wherein the computer storage medium stores a computer program, and when the computer program is executed by a computer, the method according to any one of claims 21 to 25 is implemented.

Intelligent control apparatus 100

| People | Detection and heating circuit 110 | Control circuit 120 | Transceiver circuit 130 |

Target device 200

Transceiver 210

Processor 220

FIG. 1

Heating circuit 330

Detection circuit 320

Heat conducting material 312

Conducting material 311

Detection and heating material 310

FIG. 2

Detection circuit
320'

Heating circuit
330'

Detection and heating
material 310'

FIG. 3

Detection circuit 400

Direct current or
working frequency
suppression module 410

Signal amplification
module 420

Analog filtering
module 430

Digital filtering
module 450

Digital-to-analog
conversion module
440

FIG. 4

Fatigue category

Output layer 540

Fully connected layer 530

Hidden layer n (53n)

Hidden layer 2 (532)

Hidden layer 1 (531)

Convolutional layer or pooling layer 520

526

525

524

523

522

521

Convolutional neural network (CNN) 500

Input layer 510

Signal feature of a bioelectric signal

FIG. 5

| A control circuit instructs a detection and heating circuit to set a working mode of a detection and heating material to a detection mode | 601 |

| Obtain first detection information of a user, where the first detection information is obtained based on a first bioelectric signal of the user | 602 |

| A transceiver circuit sends, to a target device, second detection information corresponding to the first detection information, where the second detection information is obtained based on the first detection information | 603 |

| Receive first instruction information sent by the target device and corresponding to the second detection information | 604 |

| Switch the working mode of the detection and heating material to a heating mode according to the first instruction information, and control duration and/or a heating temperature of the detection and heating material working in the heating mode | 605 |

FIG. 6

A control circuit instructs a detection and heating circuit to set a working mode of a detection and heating material to a detection mode — 701

A detection and heating circuit obtains a third bioelectric signal of a user by using the detection and heating material, and processes the obtained third bioelectric signal to obtain fifth detection information corresponding to the third bioelectric signal — 702

A transceiver circuit sends, to a target device, sixth detection information corresponding to the fifth detection information, where the sixth detection information is obtained based on first detection information — 703

The target device receives the sixth detection information — 704

The target device trains a user state category classification model based on the sixth detection information — 705

FIG. 7

| | |
|---|---|
| Set a first preset value of duration of a heating mode of a detection and heating material | ∽ 801 |

| | |
|---|---|
| Switch a working mode of the detection and heating material to a detection mode when heating duration of the detection and heating material is greater than or equal to the first preset value | ∽ 802 |

| | |
|---|---|
| A detection and heating circuit obtains a second bioelectric signal of a user by using the detection and heating material, and processes the obtained second bioelectric signal to obtain third detection information corresponding to the second bioelectric signal | ∽ 803 |

| | |
|---|---|
| Send, to a target device, fourth detection information corresponding to the third detection information | ∽ 804 |

| | |
|---|---|
| The target device receives the fourth detection information | ∽ 805 |

| | |
|---|---|
| The target device updates a user state category classification model based on the fourth detection information | ∽ 806 |

FIG. 8

Apparatus 900

People — Detection module 930 — Control module 910 — Transceiver module 940 — Target device

Heating/massage module 920

FIG. 9

Temperature controller 1004

Analog switch group 1005

Control logic transmitter 1006

Detection and heating material 1001

Bioelectric processor 1007

Heating circuit 1003

Power supply 1002

FIG. 10

Intelligent control apparatus 100'

Control circuit 120'

People

Detection and heating circuit 110'

Mode control module 121'

Policy module 124'

Processing module 122'

Analysis module 123'

FIG. 11

| Control a detection and heating material to work in a detection mode | 1201 |
|---|---|

| Obtain first detection information of a user | 1202 |
|---|---|

| Switch a working mode of the detection and heating material to a heating mode based on the first detection information, and control duration and/or a heating temperature of the detection and heating material working in the heating mode | 1203 |
|---|---|

FIG. 12

| A control circuit instructs a detection and heating circuit to set a working mode of a detection and heating material to a detection mode | 1301 |
|---|---|

| The detection and heating circuit obtains a third bioelectric signal of a user by using the detection and heating material, and processes the obtained third bioelectric signal to obtain a digital third bioelectric signal | 1302 |
|---|---|

| Perform processing such as decomposition and feature extraction on the digital third bioelectric signal, and obtain a feature signal of the third bioelectric signal | 1303 |
|---|---|

| Train a user state category classification model based on the feature signal | 1304 |
|---|---|

FIG. 13

| Set a first preset value of duration of a heating mode of a detection and heating material | ～ 1401 |

| Switch a working mode of the detection and heating material to a detection mode when heating duration of the detection and heating material is greater than or equal to the first preset value | ～ 1402 |

| A detection and heating circuit obtains a second bioelectric signal of a user by using the detection and heating material, and processes the obtained second bioelectric signal to obtain a digital second bioelectric signal | ～ 1403 |

| A control circuit updates a user state category classification model based on the digital second bioelectric signal | ～ 1404 |

FIG. 14

| Temperature controller 1504 | Analog switch group 1505 | Heating circuit 1502 |
| Control logic transmitter 1509 | Bioelectric processor 1506 |
| Detection and heating material 1501 | Memory 1507 |
| Temperature adjustment processor 1508 |
| Power supply 1503 |

FIG. 15

Intelligent control
apparatus 1620

Target device

1621

Control
circuit 1622

1623

1624

Eye mask
body 1610

FIG. 16

**INTERNATIONAL SEARCH REPORT**

| International application No. |
|---|
| **PCT/CN2019/130792** |

### A.    CLASSIFICATION OF SUBJECT MATTER

A61H 7/00(2006.01)i;  A61F 7/00(2006.01)i

According to International Patent Classification (IPC) or to both national classification and IPC

### B.    FIELDS SEARCHED

Minimum documentation searched (classification system followed by classification symbols)

A61H,  A61F,  A61B

Documentation searched other than minimum documentation to the extent that such documents are included in the fields searched

Electronic data base consulted during the international search (name of data base and, where practicable, search terms used)

CNPAT, EPODOC, WPI, CNKI: 华为, 杨晖, 张慧敏, 刘畅, 李旭, 可穿戴, 智能, 控制, VR, 眼镜, 眼罩, 头盔, 头带, 头罩, 手环, 指套, 面罩, 生物电信号, 生理信号, 脑电, 肌电, 眼电, 心电, 加热, 刺激, 温度, 振动, 按摩, 模式, 功能, 集成, 合并, 兼具, 切换, 变化, intelligent w control+, wearable, virtual w reality, glasses, helmet, eyepatch, headband, band, smart w bracelet, bioelectrical w signal, physiological w signal, electroencephalogram, EEG, electromyogram, EMG, electrooculography, EOG, electrocardiograph, ECG, heat+, temperature, massage, vibrat+, stimulat+, chang+, mode?, integrat+, switch+

### C.    DOCUMENTS CONSIDERED TO BE RELEVANT

| Category* | Citation of document, with indication, where appropriate, of the relevant passages | Relevant to claim No. |
|---|---|---|
| Y | CN 208784232 U (SHENZHEN H&T DATA RESOURCE AND CLOUD TECHNOLOGY CO., LTD.) 26 April 2019 (2019-04-26)<br>  description paragraphs 0002]-0040, figures 1-2 | 1-27 |
| Y | CN 106390280 A (WINBOND ELECTRONICS CORPORATION) 15 February 2017 (2017-02-15)<br>  description, paragraphs 0002-0036, figures 1-3 | 1-27 |
| A | CN 202456505 U (AC CARPI APPAREL COMPANY LIMITED) 03 October 2012 (2012-10-03)<br>  entire document | 1-27 |
| A | CN 104784008 A (QIANHAI ANYCHECK INFORMATION TECHNOLOGY COMPANY et al.) 22 July 2015 (2015-07-22)<br>  entire document | 1-27 |
| A | CN 206453982 U (GUANGZHOU UNIVERSITY) 01 September 2017 (2017-09-01)<br>  entire document | 1-27 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 September 2020** | **29 September 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/130792** |

**C.     DOCUMENTS CONSIDERED TO BE RELEVANT**

| Category* | Citation of document, with indication, where appropriate, of the relevant passages | Relevant to claim No. |
| --- | --- | --- |
| A | CN 205139861 U (SHENZHEN QIANHAI ANYCHECK INFORMATION TECHNOLOGY COMPANY) 06 April 2016 (2016-04-06)<br>  entire document | 1-27 |
| A | CN 104799837 A (SHANGHAI PHICOMM COMMUNICATION CO., LTD.) 29 July 2015 (2015-07-29)<br>  entire document | 1-27 |
| A | US 2019275328 A1 (SETPOINT MEDICAL CORPORATION) 12 September 2019 (2019-09-12)<br>  entire document | 1-27 |

Form PCT/ISA/210 (second sheet) (January 2015)

<div style="text-align:center">

**INTERNATIONAL SEARCH REPORT**

Information on patent family members

</div>

International application No.

**PCT/CN2019/130792**

| Patent document cited in search report | | | Publication date (day/month/year) | Patent family member(s) | | | Publication date (day/month/year) |
|---|---|---|---|---|---|---|---|
| CN | 208784232 | U | 26 April 2019 | US | 2017043159 | A | 15 February 2017 |
| CN | 106390280 | A | 15 February 2017 | | None | | |
| CN | 202456505 | U | 03 October 2012 | | None | | |
| CN | 104784008 | A | 22 July 2015 | | None | | |
| CN | 206453982 | U | 01 September 2017 | | None | | |
| CN | 205139861 | U | 06 April 2016 | WO | 2017080032 | A1 | 18 May 2017 |
| CN | 104799837 | A | 29 July 2015 | CN | 104799837 | B | 31 July 2018 |
| US | 2019275328 | A1 | 12 September 2019 | | None | | |

Form PCT/ISA/210 (patent family annex) (January 2015)